# EUROPEAN PATENT SPECIFICATION

(11) **EP 1 954 838 B1**
(45) Date of publication and mention of the grant of the patent: **26.02.2014**
(21) Application number: 06850753.2
(22) Date of filing: 14.11.2006
(51) Int. Cl.: C12Q 1/68

(54) **CODED MOLECULES FOR DETECTING TARGET ANALYTES**
KODIERTE MOLEKÜLE ZUR ERKENNUNG VON ZIELANALYTEN
MOLECULES CODEES PERMETTANT DE DETECTER DES SUBSTANCES CIBLES A ANALYSER

(30) Priority: 14.11.2005 US 736960 P
(43) Date of publication of application: 13.08.2008
(73) Proprietor: Life Technologies Corporation, Carlsbad, CA 92008 (US)
(72) Inventor: LIVAK, Kenneth J., San Jose, CA 95117 (US)
(74) Representative: Wöhler, Christian
(86) International application number: PCT/US2006/060890
(87) International publication number: WO 2007/120265

(56) References cited:
- EP-A2- 1 544 310
- US-A1- 2002 142 344
- US-A1- 2003 141 189
- US-A1- 2003 211 502
- US-A1- 2004 166 505
- US-A1- 2005 069 895
- US-B1- 6 600 026

## Description

### 1. CROSS REFERENCE TO RELATED APPLICATIONS

This application claims benefit under 35 U.S.C. §119(e) to U.S. application Serial No. 60/736,960, filed November 14, 2005.

### 2. INTRODUCTION

Analyte detection for purposes of diagnostics and screening has focused on miniaturization and multiplexing to broaden the analytes detectable in a single assay, increase sensitivity of detection, and decrease the sample size. Various assay formats are available for detecting many different analytes in small sample volumes.

Bead based systems use microparticles containing probes that bind to a specific target analyte. To identify a bead and its associated target specific probe, the microparticles have an identifiable set of characteristics, also called signatures or codes, that allows one bead to be distinguished from another bead. Some bead systems use an optical signature based on different fluorophores while other bead systems rely on different physical characteristics, such as size, shape, and surface features. Engraving patterns onto the microparticles increases the number of codes available to distinguish one microparticle from another microparticle (see, e.g., U.S. Published Application Nos. 2002/0084329 and 2003/0153092). For detection, microparticles can be randomly assembled into wells or cavities and detected optically by scanning microscopy or fiber optic based image analysis. Individual beads are also detectable by flow cytometry techniques developed for cell sorting procedures (Goodey et al., 2001, J Am Chem Soc 123(11):2559-70). The small size of the microparticles coupled with the ability to characterize each bead and associate it with a specific target analyte allows this format to be designed for high throughput analysis of specific DNAs (*e.g.,* single nucleotide polymorphisms), RNAs (*e.g.,* transcripts and splice variants), and proteins (*e.g.,* disease specific antibodies).

Another array format is the high-density microarray in which probes for detecting a target analyte are attached to a substrate in a two dimensional pattern. Each attachment area or probe cell contains a probe that binds a different target analyte. Identifying the presence of a specific target analyte relies on a signal generated upon binding of the target analyte to a specific probe and the spatial location (*i.e.,* address) of the signal on the two dimensional pattern. The size of a probe cell determines the number of probes that can be attached to the substrate, and thus is determinative of the number of analytes detectable in a single reaction. Various techniques to reduce the size of the probe cell include photolithography techniques and digital micromirror systems (Singh-Gasson et al., 1999, Nat Biotechnol 17:974-978). Arrays with probe cell densities of 3 x 10⁴ to 4 x 10⁵ per array (*e.g.,* 300,000 probe cells in an area of 1.2 cm²) have been used for gene expression profiling and single nucleotide polymorphism detection.

A different approach than arrays is the use of electrophoretic tags that differ in the charge to mass ratio (see, *e.g.,* Tian et al., 2004, Nucleic Acids Res. 32(16):e126; U.S. Patent No. 6,818,399; and U.S. Patent No. 6,682,887). Electrophoretic tags (*e.g.,* eTags®) are typically modified fluorescent molecules separable by capillary electrophoresis based on differences in their charge to mass ratio. The tags are attached to various ligands specific to the analyte of interest, for example, nucleic acid probes and antibodies. After mixing a sample with the tags, a molecular scissor is activated to release the mobility-modified fluorescent tags from the ligands bound to the target analyte. The released mobility-modified fluorescent dyes are detected via capillary electrophoresis to determine the type and abundance of analytes present in the sample. The number of specific targets detectable by electrophoretic tags is dependent on the availability of different fluorophores and the ability to separate the mobility-modified fluorophores from one another.

Although the multiplexing system in the formats currently practiced have the capability of detecting a large number of different target analytes, these formats have a number of disadvantages. These include, among others, the presence of surface effects that slows the kinetics of interaction between target analyte and probe, presence of non-specific interactions with a substrate surface, and reliance on summing of signals from a population of probe/analyte interactions. These factors place limits on assay speed, specificity, and sensitivity. Thus, it is desirable to develop alternative methods that have high sensitivity and obviates or reduces the effect of surfaces on target/analyte interactions.

### 3. SUMMARY

The present disclosure provides methods of detecting a target analyte by translocating a coded molecule through a nanopore, where the coded molecule comprises an ordered plurality of code regions. One or more of the code regions is non-single-stranded and each code region has a detectable property such that detecting the ordered code regions generates a defined signal pattern. In some embodiments, the coded molecule can have a plurality of non-single-stranded coded regions. The coded molecule can further comprise a moiety capable of binding to a target analyte. For detection, the coded molecule is translocated through a nanopore that is dimensioned for passage of the non-single-stranded region and each code region scanned or interrogated to generate the defined signal pattern. Relating the signal pattern of the scanned coded molecule to the presence of a specific binding moiety allows determining the presence of a specific target analyte. In some embodiments, the method can further comprise detecting the presence of the target analyte bound to the moiety. Detectable properties of the code regions include, among others, current blockade, electron tunneling current, charge-induced field effect, nanopore transit time, optical signal, light scattering, and plasmon resonance.

In other aspects, the method of detecting a target analyte comprises translocating through a nanopore a coded molecule of a population of coded molecules, wherein the population of coded molecules comprises at least a first and second subpopulation and each subpopulation generates a defined signal pattern that is distinguishable between the first and second subpopulations. The first subpopulation can comprise a first moiety capable of binding to a first target analyte and the second subpopulation comprises a second moiety capable of binding to a second analyte, where the first and second binding moieties are capable of binding to different target analytes. In some embodiments, a plurality of different target analytes can be detected by using a plurality of coded molecule subpopulations in which each subpopulation generates a defmed signal pattern distinguishable in the plurality of subpopulations and comprises a binding moiety that binds a target analyte different from those bound by the binding moieties present on the other subpopulations.

The disclosure further provides methods of making the coded molecules by segregating mixtures of scaffolds and nucleobase oligomers in reaction compartments. Reaction compartments include, among others, microcapsule preparations, inverse emulsions, and aqueous slugs formed in capillary channels. In some embodiments, the method of forming the coded molecules comprises contacting a reaction compartment with one or more nucleobase oligomers, wherein the reaction compartment comprises a single-stranded nucleobase scaffold and each nucleobase oligomer hybridizes to a defined sequence on the scaffold to form a non-single-stranded code region. Different combinations of nucleobase oligomers can be used to generate different coded molecules in each reaction compartment. Combinations of nucleobase oligomers in which one or more nucleobase oligomers are different between combinations are useful for forming different coded molecules from identical scaffolds.

In some embodiments, the method of forming the coded molecule comprises contacting a population of microcapsules with a first nucleobase oligomer, wherein the microcapsules comprise a single-stranded nucleobase scaffold. The first nucleobase oligomer hybridizes to a first defined sequence on the scaffold to form a first non-single-stranded code region. This population of microcapsules is then used to generate at least a first and second subpopulation. The first subpopulation is contacted with a second nucleobase oligomer and the second subpopulation contacted with a third nucleobase oligomer, wherein the second nucleobase oligomer hybridizes to a second defined sequence and the third nucleobase oligomer hybridizes to a third defined sequence on the scaffold to form a second non-single-stranded code region in each subpopulation. In some embodiments, the second and third nucleobase oligomers can be the same or different type of nucleobase polymers (*e.g.,* PNA and DNA). In other embodiments, the second and third defined sequences can be the same or different sequences on the single-stranded scaffold. Repeating the steps of forming microcapsule subpopulations and hybridizing nucleobase oligomers can be used to generate additional non-single stranded code regions on the scaffolds.

In other embodiments, the method of forming the coded molecules can comprise contacting a population of microcapsules with a first nucleobase oligomer, wherein the microcapsules comprise a single-stranded nucleobase polymer scaffold, and the first nucleobase oligomer hybridizes to a first defined sequence on the scaffold to form a first non-single-stranded code region. The population of microcapsules is used to generate a plurality of microcapsule subpopulations, and each subpopulation contacted with a second nucleobase oligomer that hybridizes to a second defined sequence on the scaffold to form a second non-single-stranded code region. In some embodiments, the first or second nucleobase oligomer used in each subpopulation comprises a different nucleobase polymer, thereby providing a basis to distinguish the first subpopulation from the second subpopulation of coded molecules. Repeating the steps of forming microcapsule subpopulations and hybridizing nucleobase oligomers can be used to generate a plurality of non-single stranded code regions on the scaffolds and ultimately coded molecules that generate defined signal patterns distinguishable from other coded molecules.

The methods for making the coded molecules can further comprise crosslinking the hybridized nucleobase oligomer to the scaffold, for example, with an interstrand crosslinking agent. Crosslinking can be carried out after hybridization of each nucleobase oligomer or following hybridization of all the nucleobase oligomers to the scaffold. Crosslinking stabilizes the non-single-stranded code regions to the conditions for sample processing and translocation through the nanopore.

The coded molecules can be used to detect a variety of analytes, including, among others, small organic molecules, peptides and proteins, nucleic acids, oligosaccharides, steroids, and pathogenic organisms. In some embodiments, the coded molecules are used to detect genetic polymorphisms, including variations resulting from nucleotide substitutions, insertions, and deletions. Genetic abnormalities can also be detected using the methods herein.

Further provided are kits comprising the coded molecules for detecting target analytes. The kits can comprise a single type of coded molecule for detecting a single target analyte or different coded molecules for detecting the presence of multiple target analytes. Kits can also include nanopore devices, representative coded molecules with identifiable signal patterns, and instructions for using the kits.

### 4. BRIEF DESCRIPTION OF THE DRAWINGS

The skilled artisan will understand that the drawings, described below, are for illustration purposes only. The drawings are not intended to limit the scope of the present teachings in any way.

FIG. 1 is an illustration of a coded molecule formed on a linear single-stranded nucleobase polymer scaffold. Non-single-stranded code regions are formed by hybridizing nucleobase polymers (e.g., oligonucleotides) onto defined positions on the scaffold. Any number of code regions can be created on a scaffold to generate large numbers of coded molecules with unique signal patterns.

FIG. 2 is an illustration of distinguishable coded molecules formed by changing the order of the code regions on a nucleobase polymer scaffold. Changing the order of code regions can produce different signal patterns.

FIG. 3 is an illustration of distinguishable coded molecules formed by changing the type of nucleobase polymer used to form one of the non-single-stranded code region. Although the scaffold can be identical and the nucleobase oligomers hybridize to the same sequences on each of the defined code regions of the scaffold, one of the non-single-stranded code regions formed differ in the type of nucleobase polymer hybridized to the scaffold. This difference provides a basis to distinguish one coded molecule from the other.

FIG. 4 is an illustration of a coded molecule formed by hybridizing together segments of nucleobase polymers having overlapping complementary regions, thereby forming a single coded molecule. In the illustrated embodiment, the hybridized regions form the non-single stranded code regions.

FIG. 5 is an illustration of a method of forming coded molecules in reaction compartments generated as inverse emulsions or microcapsules.

FIG. 6 is an illustration of a method of forming coded molecules using a capillary channel in which are formed reaction compartments of aqueous slugs separated by an immiscible liquid.

FIG. 7 is an illustration of a method of forming coded molecules in a capillary channel by sequential addition of nucleobase oligomers into aqueous slugs comprising single-stranded scaffolds.

### 5. DETAILED DESCRIPTION

It is to be understood that both the foregoing general description, including the drawings, and the following detailed description are exemplary and explanatory only and are not restrictive of this disclosure. In this disclosure, the use of the singular includes the plural unless specifically stated otherwise. Also, the use of "or" means "and/or" unless stated otherwise. Similarly, "comprise," "comprises," "comprising" "include," "includes," and "including" are not intended to be limiting.

It is to be further understood that where descriptions of various embodiments use the term "comprising," those skilled in the art would understand that in some specific instances, an embodiment can be alternatively described using language "consisting essentially of" or "consisting of."

The section headings used herein are for organizational purposes only and not to be construed as limiting the subject matter described.

### 5.1 Definitions and Terms

As used throughout the instant application, the following terms shall have the following meanings:

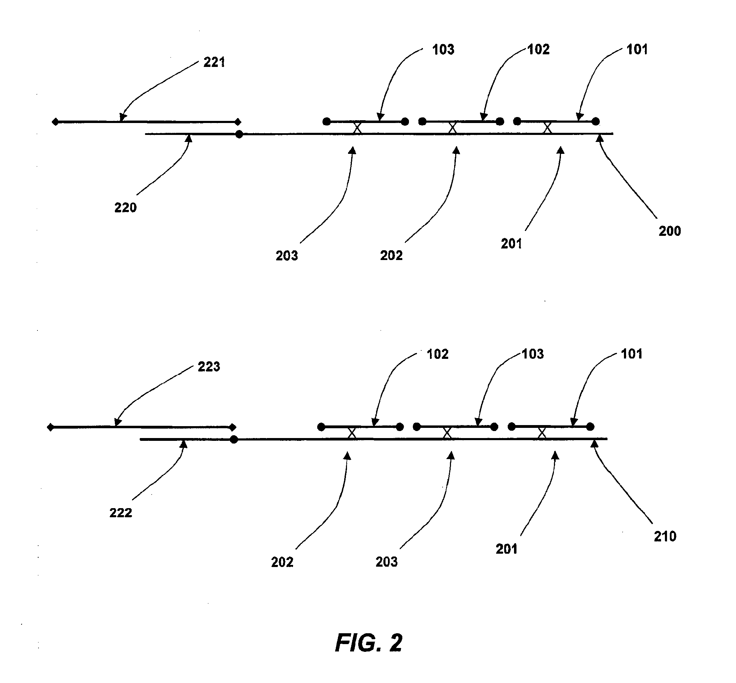

"Nucleobase" or "Base" means those naturally occurring and synthetic heterocyclic moieties commonly known in the art of nucleic acid or polynucleotide technology or polyamide or peptide nucleic acid technology for generating polymers that can hybridize to polynucleotides in a sequence-specific manner. Non-limiting examples of suitable nucleobases include: adenine, cytosine, guanine, thymine, uracil, 5-propynyl-uracil, 2-thio-5-propynyl-uracil, 5-methylcytosine, pseudoisocytosine, 2-thiouracil and 2-thiothymine, 2-aminopurine, N9-(2-amino-6-chloropurine), N9-(2,6-diaminopurine), hypoxanthine, N9-(7-deaza-guanine), N9-(7-deaza-8-aza-guanine) and N8-(7-deaza-8-aza-adenine). Other non-limiting examples of suitable nucleobases include those nucleobases illustrated in Figures 2(A) and 2(B) of Buchardt et al. (W0 92/20702 or W0 92/20703). Nucleobases can be linked to other moieties to form nucleosides, nucleotides, and nucleoside/tide analogs.

"Nucleoside" refers to a compound having a purine, deazapurine, or pyrimidine nucleoside base, e.g., adenine, guanine, cytosine, uracil, thymine, 7-deazaadenine, 7-deazaguanosine, that is linked to the anomeric carbon of a pentose sugar at the 1' position, such as a ribose, 2'-deoxyribose, or a 2',3'-di-deoxyribose. When the nucleoside base is purine or 7-deazapurine, the pentose is attached at the 9-position of the purine or deazapurine, and when the nucleoside base is pyrimidine, the pentose is attached at the 1-position of the pyrimidine (*see, e.g.,* Kornberg and Baker, 1992, *DNA Replication,* 2nd Ed., Freeman). The term "nucleotide" as used herein refers to a phosphate ester of a nucleoside, e.g., a mono-, a di-, or a triphosphate ester, wherein the most common site of esterification is the hydroxyl group attached to the C-5 position of the pentose. "Nucleotide 5'-triphosphate" refers to a nucleotide with a triphosphate ester group at the 5' position. The term "nucleoside/tide" as used herein refers to a set of compounds including both nucleosides and/or nucleotides.

"Nucleobase Polymer" or "Nucleobase Oligomer" refers to two or more nucleobases that are connected by linkages that permit the resultant nucleobase polymer or oligomer to hybridize to a polynucleotide having a complementary nucleobase sequence. Nucleobase polymers or oligomers include, but are not limited to, poly- and oligonucleotides (*e*.*g*., DNA and RNA polymers and oligomers), poly- and oligonucleotide analogs and poly- and oligonucleotide mimics, such as polyamide or peptide nucleic acids. Nucleobase polymers or oligomers can vary in size from a few nucleobases, from 2 to 40 nucleobases, to several hundred nucleobases, to several thousand nucleobases, or more.

"Polynucleotides" or "Oligonucleotides" refers to nucleobase polymers or oligomers in which the nucleobases are connected by sugar phosphate linkages (sugar-phosphate backbone). Exemplary poly- and oligonucleotides include polymers of 2'-deoxyribonucleotides (DNA) and polymers of ribonucleotides (RNA). A polynucleotide can be composed entirely of ribonucleotides, entirely of 2'-deoxyribonucleotides or combinations thereof.

"Polynucleotide Analog" or "Oligonucleotide Analog" refers to nucleobase polymers or oligomers in which the nucleobases are connected by a sugar phosphate backbone comprising one or more sugar phosphate analogs. Typical sugar phosphate analogs include, but are not limited to, sugar alkylphosphonates, sugar phosphoramidites, sugar alkyl- or substituted alkylphosphotriesters, sugar phosphorothioates, sugar phosphorodithioates, sugar phosphates and sugar phosphate analogs in which the sugar is other than 2'-deoxyribose or ribose, nucleobase polymers having positively charged sugar-guanidyl interlinkages such as those described in U.S. Patent No. 6,013,785 and U.S. Patent No. 5,696,253 (see also, Dagani 1995, Chem Eng News 4-5:1153; Dempey et al., 1995, J Am Chem Soc 117:6140-6141). Such positively charged analogues in which the sugar is 2'-deoxyribose are referred to as "DNGs," whereas those in which the sugar is ribose are referred to as "RNGs." Specifically included within the definition of poly- and oligonucleotide analogs are locked nucleic acids (LNAs; see, *e*.*g*., Elayadi et al., 2002, Biochemistry 41:9973-9981; Koshkin et al., 1998, J Am Chem Soc 120:13252-3; Koshkin et al., 1998, Tetrahedron Letters, 39:4381-4384; Jumar et al., 1998, Bioorganic Medicinal Chemistry Letters 8:2219-2222; Singh and Wengel, 1998, Chem Commun 12:1247-1248; WO 00/56746; WO 02/28875; and, WO 01/48190 and nucleic acids with sugar-phosphates other than deoxyribose- or ribose-phosphate backbone, for example, hexopyranosyl-phosphate backbones (Eschenmoser, 1999, Science 284:2118-2124).

"Polynucleotide Mimic" or "Oligonucleotide Mimic" refers to a nucleobase polymer or oligomer in which one or more of the backbone sugar-phosphate linkages are replaced with a sugar-phosphate analog. Such mimics are capable of hybridizing to complementary polynucleotides or oligonucleotides, or polynucleotide or oligonucleotide analogs or to other polynucleotide or oligonucleotide mimics, and can include backbones comprising one or more of the following linkages: positively charged polyamide backbone with alkylamine side chains as described in U.S. Patent No. 5,786,461; U.S. Patent No. 5,766,855; U.S. Patent No. 5,719,262; U.S. Patent No. 5,539,082 and WO 98/03542 (see also, Haaima et al., 1996, Angewandte Chemie Int'l Ed in English 35:1939-1942; Lesnick et al., 1997, Nucleosid Nucleotid 16:1775-1779; D'Costa et al., 1999, Org Lett 1:1513-1516 see also Nielsen, 1999, Curr Opin Biotechnol 10:71-75); uncharged polyamide backbones as described in WO 92/20702 and U.S. Patent No. 5,539,082; uncharged morpholinophosphoramidate backbones as described in U.S. Patent No. 5,698,685, U.S. Patent No. 5,470,974, U.S. Patent No. 5,378,841 and U.S. Patent No. 5,185,144 (see also, Wages et al., 1997, BioTechniques 23:1116-1121); peptide-based nucleic acid mimic backbones (see, *e.g.,* U.S. Patent No. 5,698,685); carbamate backbones (see, *e.g.,* Stirchak and Summerton, 1987, J Org Chem 52:4202); amide backbones (see, *e.g.,* Lebreton, 1994, Synlett 1994:137); methylhydroxyl amine backbones (see, *e.g.,* Vasseur et al., 1992, J Am Chem Soc 114:4006); 3'-thioformacetal backbones (see, *e*.*g*., Jones et al., 1993, J Org Chem 58:2983); sulfamate backbones (see, *e.g.,* U.S. Patent No. 5,470,967); and α-threofuranosyl backbones (Schoning et al., Science 2901347-1351).

"Peptide Nucleic Acid" or "PNA" refers to poly- or oligonucleotide mimics in which the nucleobases are connected by amino linkages (polyamide backbone) such as described in any one or more of U.S. Patent Nos. 5,539,082, 5,527,675; 5,623,049; 5,714,331; 5,718,262; 5,736,336; 5,773,571; 5,766,855; 5,786,461; 5,837,459; 5,891,625; 5,972,610; 5,986,053; 6,107,470; 6,451,968; 6,441,130; 6,414,112; and 6,403,763. The term "peptide nucleic acid" or "PNA" shall also apply to any oligomer or polymer comprising two or more subunits of those polynucleotide mimics described in the following publications: Lagriffoul et al., 1994, Bioorg Med Chem Lett 4: 1081-1082; Petersen et al., 1996, Bioorg Med Chem Lett 6: 793-796; Diderichsen et al., 1996, Tett Lett 3 7: 475-478; Fujii et al., 1997, Bioorg Med Chem Lett 7: 637-627; Jordan et al., 1997, Bioorg Med Chem Lett 7:687-690; Krotz et al., 1995, Tett Lett 36: 6941-6944; Lagriffoul et al, 1994, Bioorg Med Chem Lett 4:1081-1082; Diederichsen, U., 1997, Bioorg Med Chem Lett 7: 1743-1746; Lowe et al., 1997, J Chem Soc Perkin Trans 1:539-546; Lowe et al., 1997, J Chem Soc Perkin Trans 11:547-554; Lowe et al., 1997, J Chem Soc Perkin Trans 1:555-560; Howarth et al., 1997, J Org Chem 62:5441-5450; Altmann et al., 1997, Bioorg Med Chem Lett 7:1119-1122; Diederichsen, U., 1998, Bioorg Med Chem Lett 8:165-168; Diederichsen et al., 1998, Angew Chem Int Ed 37:302-305; Cantin et al., 1997, Tett Lett 38:4211-4214; Ciapetti et al., 1997, Tetrahedron 53:1167-1176; Lagriffoule et al., 1997, Chem Eur J 3:912-919; Kumar et al., 2001, Org Lett 3(9):1269-1272; and the Peptide-Based Nucleic Acid Mimics (PENAMs) disclosed in WO 96/04000. Some examples of PNAs are those in which the nucleobases are attached to an N-(2-aminoethyl)-glycine backbone, *i.e.,* a peptide-like, amide-linked unit (see, *e.g.,* U.S. Patent No. 5,719,262; WO 92/20702; and Nielsen et al., 1991, Science 254:1497-1500).

"Chimeric Nucleobase Oligomer" or "Chimeric Nucleobase Polymer" refers to a nucleobase polymer or oligomer comprising a plurality of different polynucleotides, polynucleotide analogs and polynucleotide mimics. For example, a chimeric nucleobase polymer can comprise a sequence of DNA linked to a sequence of RNA. Other examples of chimeric polymer include a sequence of DNA linked to a sequence of PNA, and a sequence of RNA linked to a sequence of PNA.

"Detectable Tag" refers to a moiety that, when attached to another molecule described herein, *e.g.,* an oligonucleotide, nucleobase polymer scaffold, a target analyte, renders such molecule detectable using known detection methods, *e.g.,* spectroscopic, photochemical, electrochemiluminescent, and charge-induced field effect. A detectable tag can have one or more than one label, including different types of labels. Exemplary tags include, but are not limited to, fluorophores, radioisotopes, nanoparticles, and quantum dots. Such tags allow direct detection of labeled compounds by a suitable detector, *e*.*g*., a fluorometer.

"Watson/Crick Base-Pairing" refers to a pattern of specific pairs of nucleobases and analogs that bind together through sequence-specific hydrogen-bonds, *e*.*g*., A pairs with T and U, and G pairs with C.

Annealing" or "Hybridization" refers to the base-pairing interactions of one nucleobase polymer with another that results in the formation of a double-stranded structure, a triplex structure or a quaternary structure. Annealing or hybridization can occur *via* Watson-Crick base-pairing interactions, but can also be mediated by other hydrogen-bonding interactions, such as Hoogsteen base pairing.

"Deoxynucleotides" or "dNTPs" refer to deoxynucleoside triphosphate precursors, *i.e.,* dATP, dTTP, dGTP, and dCTP, and dUTP.

"adjacent" in the context of a code region refers to code regions that are adjoining. For instance, when the code regions are nucleobase oligomers hybridized to a single-stranded nucleobase polymer scaffold, adjacent code regions have the nucleobase oligomers separated by not more than a single nucleobase residue of the scaffold.

"Polypentide," "Peptide," and "Protein" refer to a polymer of amino acid residues and amino acid analogs in which the peptide backbone is an amide linkage. Amino acids forming the polypeptide, peptide or protein can be genetically encoded amino acids, naturally occurring non-genetically encoded amino acids, and synthetic amino acids as described herein. Polypeptide, peptide, or protein encompasses linear, branched, or cyclic forms of amino acid polymers.

"Polypeptide analog," "Peptide analog," and "Protein analog" refer to a polymer of amino acid residues or amino acid analogs in which one or more of the amide linkages is replaced by other than an amide linkage, such as a substituted amide linkage or isostere of an amide linkage. Substituted amide linkages include, but are not limited to, groups of the formula -C(O)NR', where R' can be an substituted or unsubstituted alkyl, aryl, arylalkyl, heteroaryl, or heteroarylalkyl.

Isosteres of amides linkages include, but are not limited to, -NR"-SO-, -NR"-S(O)₂-, -CH₂-CH₂-, -CH=CH- (*cis* and *trans*), -CH₂-NH-, -CH₂-S-, -CH₂-O-, -C(O)-CH₂-, -CH(OH)-CH₂- and -CH₂-S(O)₂-, where R" is hydrogen or R' as previously defined. Peptide analogs with non-amide linkages, as well as methods of synthesizing such analogs, are well-known (e.g., Spatola, 1983, "Peptide Backbone Modifications," in Chemistry and Biochemistry of Amino Acids, Peptides and Proteins, Weinstein, Ed., Marcel Dekker, New York, pp. 267-357; Morley, 1980, Trends Phanm Sci 1:463-468; Hudson et al., 1979, Int JProt Res 14:177-185 (-CH₂-NH-, -CH₂-CH₂); Spatola et al., 1986, Life Sci 38:1243-1249; Spatola, 1983, "Peptide Backbone Modifications: the Ψ [CH2S] Moiety as an Amide Bond Replacement," in Peptides: Structure and Function V, J. Hruby and D.H. Rich, eds., Pierce Chemical Co., Rockford, IL, pp. 341-344 (-CH₂ -S-); Hann, 1982, J Chem Soc Perkin Trans I 1:307-314 (-CH=CH-, *cis* and *trans*); Almquist et al., 1980, J Med Chem 23:1392-1398 (-C(O)-CH₂-); European Patent Application EP 45665; Chemical Abstracts CA 97:39405 (-CH(OH)-CH₂-); Holladay et al., 1983, Tetrahedron Lett. 24:4401-4404 (-CH(OH)-CH₂-); and Hruby, 1982, Life Sci 31:189-199 (-CH₂-S-).

"Polypeptide mimetic," "Peptide mimetic," and "Protein mimetic" refer to a polymer of amino acid residues or amino acid analogs in which one or more of the amide linkages are replaced with a peptidomimetic and/or amide mimetic moieties. Non-limiting examples of such moieties are described in Olson et al., 1993, J Med Chem 36:3039-3049; Ripka and Rich, 1998, Curr Opin Chem Biol 2:441-452; Borchardt et al., 1997, Adv Drug Deliv Rev 27:235-256 and the various references cited therein.

"Amino acid" refers to naturally occurring genetically encoded amino acids, naturally occurring non-genetically encoded amino acids, and synthetic amino acids. These amino acids can be in either the L- or D-configuration. Naturally occurring amino acids are those encoded by the genetic code, as well as those amino acids that are later modified, e.g., hydroxyproline, carboxyglutamate, and O-phosphoserine. Non-encoded and synthetic amino acids include, but are not limited to: the D-enantiomers of the genetically-encoded amino acids; α-aminoisobutyric acid; δ-aminovaleric acid; N-methylglycine; ornithine; citrulline; N-methylisoleucine; norleucine; homotyrosine; homotryptophan; homolysine; phosphoserine; phosphothreonine; homoaspartic acid; homoglutamic acid; homoalanine; norvaline; homoleucine, homovaline; homoisolencine; homoarginine; N-methylvaline; homocysteine; homoserine; hydroxyproline, and homoproline. Additional non-encoded amino acids will be apparent to those of skill in the art (*see, e.g.,* the various amino acids provided in Fasman, 1989, CRC Practical Handbook of Biochemistry and Molecular Biology, CRC Press, Boca Raton, FL, at pp. 3-70 and the references cited therein).

### 5.2 Coded Molecules

The present disclosure provides methods of detecting target analytes using coded molecules in which a signal pattern generated by a set of code regions on the coded molecule serves as an identifier for a target analyte binding probe used to detect the target analyte. Detecting the presence of a unique coded molecule and relating the molecule to a specific binding probe provides a basis for determining the presence or absence of a specific analyte in a sample. Generally, the methods for detecting a target analyte comprise translocating a coded molecule through a nanopore, where the coded molecule comprises an ordered plurality of code regions formed on a nucleobase polymer scaffold and each code region has a detectable property such that detecting the ordered code regions generates a signal pattern that is associated with each coded molecule. In the methods described herein, one or more of the code regions is a non-single-stranded region formed at a defined position on the scaffold, and the nanopore is of sufficient dimension that permits translocation of the non-single-stranded region. The signal pattern is decoded and related to the specific binding moiety present on the coded molecule, and thus the specific target analyte being detected. In some embodiments, the method further comprises detecting the target analyte present on the coded molecule. Single molecule analysis based on translocation through a nanopore can have a number of advantages. These include, among others, reducing the surface effects that slow the kinetics of binding between a target analyte and a probe when the probe is bound to a surface, reducing background noise resulting from non-specific interactions of analyte probe with the substrate surface, and increasing detection sensitivity by analyzing single molecules rather than summing of signals from a population of probe molecules.

The term "coded molecule" as used herein refers to a single polymeric molecule comprising a plurality of code regions present in an ordered pattern on a polymer scaffold. A "polymer scaffold" or "scaffold" can be a linear polymer on which are present the code regions. A discrete segment of the scaffold with an associated detectable property forms a code region. The single polymeric molecule can be a single chain polymer or be made of multiple polymers associated together to form a single polymeric molecule. In some embodiments, the coded molecule can comprise branched structures that can be translocated through detection region.

In some embodiments, the scaffold comprises a single chain of a nucleobase polymer capable of hybridizing to other nucleobase polymers in a sequence-specific manner. In these embodiments, the scaffold can be a single-stranded polymer of a polynucleotide, such as a single-stranded DNA, single-stranded RNA, or a single-stranded polynucleotide analog or mimic. In some embodiments where the scaffold comprises a polynucleotide analog or mimic, any number of nucleobase polymers having a backbone other than sugar phosphate linkages can be used. Polynucleotide analogs and mimics include those having linkages of sugar alkylphosphonates, sugar phosphoramidites, sugar alkyl- or substituted alkylphosphotriesters, sugar phosphorothioates, sugar phosphorodithioates, sugar phosphates and sugar phosphate analogs in which the sugar is other than 2'-deoxyribose or ribose, positively charged analogue "DNGs" and "RNGs"; positively charged polyamide backbone with alkylamine side chains; uncharged polyamide backbones; uncharged morpholino-phosphoramidate; peptide-based nucleic acid mimic backbones; carbamate backbones; amide backbones; methylhydroxyl amine backbones; 3'-thioformacetal backbones; sulfamate backbones and threofuranosyl backbones. Exemplary single nucleobase polymers include a glycol nucleic acid with an acyclic three carbon propyleneglycolphosphodiester backbone and α-threofuranosyl backbones (Schoning et al., *supra*), both of which can undergo Watson and Crick base pairing interactions (Zhang et al., 2004, J. Amer. Chem. Soc. Epub). Other types of nucleobase polymers will be apparent to the skilled artisan.

Scaffolds can also be chimeric nucleobase polymers, where the single-stranded nucleobase polymer comprises a plurality of different nucleobase polymers, such as different polynucleotides, polynucleotide analogs and polynucleotide mimics. Non-limiting examples of a chimeric scaffold include single-stranded polynucleotides comprising a segment of RNA and a segment of DNA, a segment of RNA and a segment of PNA, or a segment of DNA and a segment of PNA. Other chimeric scaffolds will be apparent to the skilled artisan.

In other embodiments, the scaffold comprises a composite of a single-stranded nucleobase polymer having various synthetic non-nucleobase polymers or linkers that connect segments of single-stranded nucleobase polymers. Various synthetic polymers can be used to connect polynucleotide segments together to from a linear polymer chain. Non-limiting examples of such polymers include polyethylene glycol (PEG), polystyrenes, polyacrylic acids, polyacetamides, polyphosphates, and other polymers that do not form Watson and Crick or Hoogsteen base pairs with a nucleobase polymer. The synthetic polymers can be block polymers or block copolymers. A non-limiting example of a composite scaffold is a polymer formed with a block polymer of polyethylene glycol and a polymer of deoxypolynucleotides, as described in Sanchez-Quesada et al., 2004, Angew Chem Int Ed 43:3063-3067 and Jaschke et al., 1994, Nucleic Acids Res 22(22):4810-4817. Other composite polymers of polynucleotides and non-polynucleotide polymers or linkers are described in, among others, U.S. Patent Application No. 2005/0153926; Greenberg et al., J Org Chem 66:7151-7154; and Pon and Yu, 2005, Nucleic Acids Res 33(6):1940-1948. .

In other embodiments, the scaffold is a linear molecule comprising discontinuous segments of single-stranded nucleobase polymers connected to form a linear coded molecule. Scaffolds of this type can be prepared by hybridizing polymers of single-stranded polynucleotides, polynucleotide analogs, or polynucleotide mimics that have complementary sequences at the terminal regions of the single-stranded molecules, although complementary sequences other than the terminal regions (*e.g.,* internal sequences) can also be used. Hybridizing the complementary terminal regions results in a linear molecule with a double-stranded region connecting at least two of the single-stranded polymers. By having appropriate single-stranded nucleobase polymers that hybridize at their terminal regions, a linear molecule comprised of multiple polymer segments can be formed. Single-stranded regions interspersed between the double-stranded hybridized portions provide defined positions for forming the code regions. Optionally, the hybridized regions connecting the segments of single-stranded polymers can function as the code regions. As further described below, connecting different polynucleotides, polynucleotide analogs, and polynucleotide mimics can be used to generate hybridized regions displaying different detectable properties.

In the various embodiments, the scaffold can be of any nucleobase sequence, with one or more defined sequences for forming the non-single-stranded code region. The arrangement of the scaffold sequences will be apparent to the skilled artisan. For example, a defined unique sequence can be separated by any of random nucleotide polymer or block polymer or coblock polymer of pyrimidines (*e.g.,* polyC, polyT, and polyU) and polypurines (polyA and polyG), where the defined sequence forms part of the non-single-stranded code region. In other embodiments, a defined sequence can separate discrete block polymer or coblock polymer of pyrimidines (*e.g.,* polyC, polyT, and polyU) or polypurines (polyA and polyG), where the block polymer region forms part or whole of a non-single stranded code region. Other repeat sequences, for example, dinucleotide or trinucleotide repeat sequences, *e*.*g*., -(AG)ₙ-, -(CT)ₙ, -(ATC)ₙ-, -(TTA)ₙ-, etc., can also be used in the various embodiments.

In still other embodiments, the scaffold can be any naturally occurring polynucleotide sequence onto which code regions can be formed at defined positions along the sequence of the polynucleotide. Non-limiting examples of naturally occurring sequences include, among others, bacteriophages (*e.g.,* φX174, fd, m13, λ, etc), plasmids, animal viruses (e.g., SV40), and cloned genes. As is apparent from the foregoing description, the available nucleobase polymers useful as a scaffold are numerous, and many different constructs can be made.

In the various embodiments, a plurality of code regions is present on the nucleobase polymer scaffold. As used herein, a "code region" refers to a defined region (synonymously "segment") on the coded molecule that has a detectable property. The code regions can be adjacent or separated from each other by other nucleobase or synthetic non-nucleobase polymer segments. In the coded molecules herein, one or more of the code regions is non-single-stranded. In some embodiments, the coded molecule comprises a plurality of non-single-stranded code regions. A "non-single-stranded region" refers to a discrete region on the coded molecule that is other than a single-stranded nucleobase polymer. Thus, in various embodiments, a non-single-stranded code region can be multistranded, such as double-stranded or triple-stranded structures. In some embodiments, the non-single-stranded code region is formed by hybridizing a defined nucleobase polymer, such as an oligonucleotide, oligonucleotide analog, or oligonucleotide mimetic or similar polynucleotides, polynucleotide analogs and polynucleotide mimetics to a specific sequence on the single-stranded nucleobase polymer to form a double-stranded region. Further, as noted above, non-single-stranded code regions can be formed by hybridizing a plurality of single-stranded nucleobase polymers having regions complementary to each other to form a single linear chain interspersed with one or more double-stranded regions.

In some embodiments where a single stranded region separates at least two non-single stranded code regions, the single stranded segment can be sufficiently long to provide a distinguishable temporal separation between detection of the non-single stranded code regions. In other embodiments, the single stranded segment can be sufficiently long to distinguish one non-single stranded code region from the other, for example, by the change in detected signal caused by the single stranded segment.

In various embodiments, one or more of the non-single-stranded code regions having a multistranded structure are not associated with any detectable tag, such as optical dyes, fluorophores, nanoparticles, electron transfer labels, or steric modifiers as further described below. Thus in various embodiments, the coded molecule can have one or more non-single-stranded code regions without a detectable tag. In other embodiments, up to all of the non-single-stranded code regions of the coded molecule are not associated with a detectable tag.

In various embodiments, a hybridized nucleobase polymer forming the code region is substantially complementary to the sequence on the scaffold, as further described below. Thus, in some embodiments, the hybridized nucleobase polymer is perfectly complementary to the nucleobase polymer sequence of the scaffold, while in other embodiments, the hybridized nucleobase polymer is not perfectly complementary to the nucleobase sequence of the scaffold. Consequently, in some embodiments, the non-single-stranded code region can comprise a hybridized region and an unhybridized (*i*.*e*., unpaired) region, the combination of which provides a detectable property for the code region. The unpaired region can be created on the hybridized nucleobase polymer and/or on the scaffold by the appropriate choice of sequence. The unpaired region can be a single nucleobase, 2 or more unpaired nucleobases, 3 or more unpaired nucleobases, 10 or more unpaired nucleobases, as long as the nucleobase polymer is stably hybridized to the scaffold. Where the code region has 2 or more unpaired nucleobases, the unpaired sites can be continuous, where there is only a single region of unpaired nucleobases, or discontinuous, where at least one unpaired nucleobase is separated from another unpaired nucleobase by one or more nucleobases that are complementary to the sequence on the scaffold. The unpaired regions can be positioned on the internal sequences or the terminal sequences of the code region. In some embodiments, the code region comprises a hybridized region formed by a portion of the hybridized nucleobase polymer that is complementary to the nucleobase sequence of the scaffold and an unhybridized region formed by a portion of the hybridized nucleobase polymer that is non-complementary to the scaffold nucleobase sequence.

In other embodiments, the multistranded region comprises a triple-stranded structure in which at least one of the three strands of nucleobase polymers interacts with one or more of the other strands by hydrogen bonding. Formation of the triple-stranded structure can be stabilized through Hoogsteen base-pairing, reverse Hoogsteen base-pairing or an equivalent type of base-pairing between a nucleobase polymer and the strands of a double-stranded polynucleotide. Various methods are available for generating triple-stranded structures (see, *e.g.,* Potaman, V.N., 2003, Expert Review of Molecular Diagnostics 3(4):481-496; Howard et al., 1971, J. Biol. Chem. 246 :7033; U.S. Patent No. 6,312,925; U.S. Patent No. 5,928,863; U.S. Patent No. 5,422,251; and PCT patent publications WO 94/17092 and WO 96/40711). In an exemplary method, regions of double-stranded DNA with purine bases predominantly on one strand and pyrimidine bases on the other strand can bind oligonucleotides of an appropriate sequence to form triple-stranded structures. Oligonucleotide analogs and mimics, such as nucleobase polymers with modified sugar residues, for example 2'-O-methylribose or peptide nucleic acid backbones, can also form stable complexes with duplex DNA to generate a triple-stranded nucleic acid (see, *e*.*g*., Sun et al., 1993, Curr Opin Struct Biol 3:345-356). In addition, modified bases or base analogues, for example 8-oxo-adenosine, pseudoisocytidine, 5-methyl cytidine, inosine, 2-aminopurine and various pyrrolo- and pyrazolopyrimidine derivatives capable of forming Hoogsteen and reverse Hoogsteen base pairs can be used. Specific parts of the double-stranded region can be a target for triple strand formation. In some embodiments, targeting the triple strand formation to the internal portion of a double-stranded structure leads to formation of a D-loop by the displaced portion of the double-stranded nucleic acid. Use of nucleobase oligomers that have a higher *Tₘ* with the scaffold than the region displaced can lead to formation of D-loop structures. D-loops are readily formed by strand displacement using PNA oligonucleotides (Peffer et al., 1993, Proc Natl Acad Sci USA. 90(22):10648-52). In other embodiments, the triple-stranded structure is created by targeting an oligonucleotide to the terminal portion(s) of a double-stranded region. The third oligonucleotide displaces a strand, generating a code region with a portion having a double-stranded structure and a portion having a single-stranded structure. In these embodiments, the non-single-stranded code region comprises a first and second oligonucleotides hybridized to the single-stranded nucleic acid, with a portion of the first and second oligonucleotides overlapping in sequence such that hybridization of the second oligonucleotide displaces the overlapping region on the first oligonucleotide. The displaced portion is generally single-stranded. The length of the triple-stranded region is generally dependent on the stability of the triple-stranded structure, and thus can be typically about 8 nucleobases or more, about 10 nucleobases or more, about 15 nucleobases or more, about 20 nucleobases or more, about 50 nucleobases or more, or about 100 nucleobases or more, up to a thousand bases or more.

In still other embodiments, the multistranded region has four or more strands in the non-single-stranded code region. Exemplary structures of these types are described in various references, for example, Bukanov et al., 1998, Proc Natl Acad Sci USA 95:5516-5520 and Phan et al., 2005, Proc Natl Acad Sci USA 18:102(3):634-9. An exemplary method for generating a multistranded structure with greater than three strands is to form a stable D-loop structure followed by hybridization of an oligonucleotide complementary to the displaced strand. Another exemplary method uses homopyrimidine PNAs, which are known to form an internal PNA₂-DNA triplex structure that results in a displaced single strand. This PNA mediated quadruplex structure is characterized by a triple-stranded structure and a single-stranded D-loop. Connecting two suitable PNAs with a linker increases the stability of the triple-stranded structure, thereby allowing facile formation of quadruplex stranded code regions. A five stranded (i.e., pentaplex) structures can be generated when an oligonucleotide is hybridized to the displaced strand of the four-stranded structure (see, *e.g.,* Eghom et al., 1995, Nucleic Acids Res. 23:217-222). Various quadruplex structures, also referred to as G4 or tetrahelical structures, have been described for telomere sequences (Iulian et al., 2005, Nucleic Acids Res. 33(6):2022-2031). Both anti-parallel and parallel structures form under defined conditions.

In the various embodiments above, a multistranded code region can comprise strands of the same type of nucleobase polymer or comprise strands of different nucleobase polymers. Non-limiting examples of a multistranded code region comprised of a single type of nucleobase polymer is one in which all the strands are DNA, RNA, or PNA. Non-limiting examples of a multistranded code region comprised of different nucleobase polymers include those in which one of the strands is selected from DNA, RNA, and PNA and is a different nucleobase polymer than at least one of the other strands. Exemplary double-stranded code region comprised of different nucleobase polymers include, among others, mixtures of DNA and RNA, mixtures of DNA and PNA, or mixtures of RNA and PNA. Various combinations of nucleobase polymers (*e.g.,* oligonucleotides, oligonucleotide analogs, and oligonucleotide mimics) are contemplated for the multistranded code regions.

In still other embodiments where the coded molecule comprises a plurality of multistranded code regions, each of the non-single-stranded code regions can comprise a combination of nucleobase polymers in which the combination is different between each non-single stranded code region. Thus, in some embodiments, the coded molecule comprises at least a first non-single-stranded code region with a first nucleobase polymer and a second non-single-stranded code region with a second nucleobase polymer, where the first and second nucleobase polymers are different types of nucleobase polymers. Non-limiting examples of these embodiments include a coded molecule with a first non-single-stranded code region in which at least one strand is a polynucleotide (*e.g.,* DNA or RNA) and a second non-single-stranded code region in which at least one strand is a polynucleotide analog or polynucleotide mimetic (*e.g.,* peptide nucleic acids and morpholino polynucleotide analogs). As will be apparent to the skilled artisan, a coded molecule with a plurality of non-single-stranded code regions can be designed to have different types of nucleobase polymers on various code regions to impart different detectable properties to each code region. Generating these types of coded molecule can proceed by hybridizing different types of nucleobase polymers to the defined code regions of a scaffold or by using a scaffold having segments formed of different nucleobase polymers.

In still other embodiments, where a plurality of non-single-stranded code regions is present on the coded molecule, the plurality of non-single-stranded code regions comprises a combination of different multistranded structures. For instance, the plurality of non-single stranded code regions can comprise at least one double-stranded code region and at least one triple-stranded code region. In other embodiments, the combination of code regions can comprise at least one double-stranded code region and at least one four-stranded code region. The combinations contemplated are numerous and will be apparent to the skilled artisan.

In the embodiments of the coded molecules herein, the plurality of coded regions is ordered on the scaffold such that detecting the detectable property generates a defined signal pattern. "Ordered" as used herein refers to a specified spatial arrangement of code regions on the scaffold. Any number of code regions can be arranged in various permutations on the scaffold to generate a large number of coded molecules. For example, code regions with the same detectable property can be positioned at different distances from each other to generate coded molecules in which the variation in time period between detectable properties of the code region allows differentiating one coded molecule from another coded molecule. In other variations, a set of code regions can be rearranged in different spatial arrangements on the scaffold to generate a large number of coded molecules based on a limited number of code regions.

In other embodiments, the plurality of code regions is ordered on the scaffold to form a coded molecule with a symmetric signal pattern. A symmetric signal pattern refers to a generated signal pattern that is the substantially identical when the coded molecule is translocated through the nanopore beginning from either end of the coded molecule. In still other embodiments, the plurality of the code regions is ordered on the single-stranded nucleic acid scaffold to form a coded molecule with an asymmetric signal pattern. An asymmetric signal pattern refers to a generated signal pattern that is not substantially identical when the coded molecule enters the pore at one end of the molecule as compared to the generated signal pattern when the molecule enters the pore from the other end. Thus, a signal pattern that is asymmetrical allows distinguishing the polarity of the coded molecule translocated through the nanopore.

The coded molecules described herein can comprise a single homogeneous population of coded molecules, where the ordered plurality of code regions is identical for each member of the population. The generated signal pattern detected upon translocation through the nanopore is substantially identical, if not identical, for each coded molecule. Slight variations in the generated signal pattern for a homogeneous population of coded molecules can arise from slight differences in the position of the coded molecule in the nanopores, such as that occurring because of Brownian motion. Subtraction of such "background noise" can reduce the amount of variation in signal pattern detected for the same coded molecule. Analysis of a homogeneous population of coded molecules is useful in detecting the presence of a single analyte or for assessing the variation in signal pattern generated when determining a "reference signal" profile for that coded molecule.

In other embodiments, the population of the coded molecules is heterogeneous, and comprises a plurality of subpopulations, where each subpopulation comprises an ordered plurality of code regions that is different from those of the other subpopulations, thereby generating a defined signal pattern characteristic for each subpopulation. As such, differences in the code regions between each subpopulation results in differences in the detected signal pattern, which allows the subpopulations to be distinguished from each other. By attaching a binding moiety that binds to a different target analyte for each subpopulation, different target analytes can be detected. Thus, a plurality of subpopulations of coded molecules is useful for detecting a plurality of different target analytes in a single sample. Different coded molecules can be obtained by using different scaffolds (*e.g.,* different scaffold sequences) or by generating distinguishable combinations of code regions, including single-stranded as well as non-single stranded code regions, on an identical scaffold, as described herein.

In accordance with the above, in some embodiments, the population of coded molecules comprises at least a first and second subpopulation, wherein one or more of the code regions are different between the subpopulation of coded molecules to produce a defined signal pattern distinguishable between the first and second subpopulation. The first subpopulation comprises a first moiety, where the first moiety is capable of binding to a first target analyte, and the second subpopulation comprises a second moiety, where the second moiety binds to a second target analyte. Generally, the first moiety of the first subpopulation and the second moiety of the second subpopulation are different such that the first and second subpopulation of coded molecules detects different target analytes.

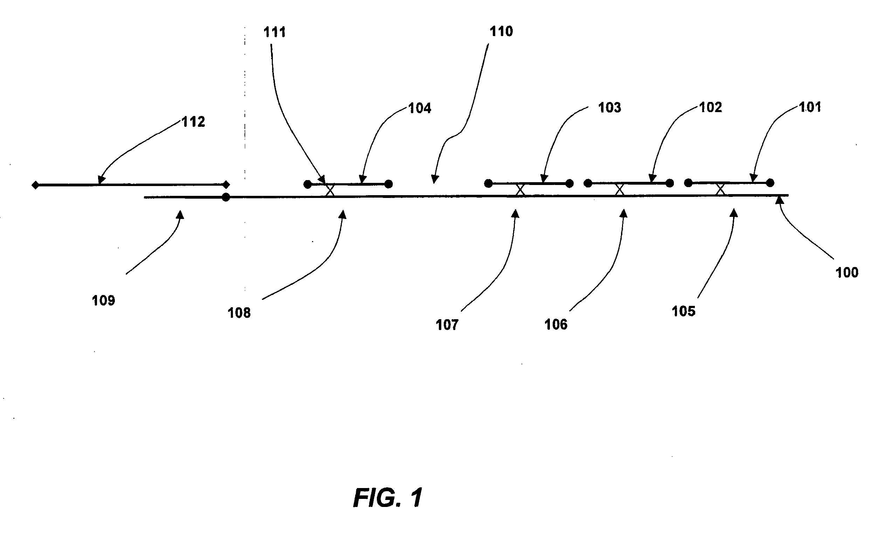

An illustration of a coded molecule is given in **FIG. 1****.** A single-stranded linear polynucleotide **100** is the basic structure for the scaffold, where the non-single-stranded code regions comprise double-stranded regions present at defined positions on the scaffold. A first nucleobase oligomer **101** comprising a DNA is hybridized to a first defmed position of the scaffold to form a first code region **105.** A second nucleobase oligomer **102,** structurally and/or chemically different from the first nucleobase oligomer, in this case a PNA, is hybridized to a second defined position on the scaffold to form a second code region **106.** A third nucleobase oligomer **103,** a PNA with a positive charge, is hybridized to a third defined position on the scaffold to form a third code region **107.** The nucleobase oligomers **101, 102,** and **103** can be positioned to be adjacent to one another, such that an entirely double-stranded region is formed on the scaffold. Any *n* number of nucleobase oligomers **104** can be added to the scaffold to form a diverse set of ***n*** code regions **108.** A part of the single-stranded nucleic acid scaffold can serve as a code region and/or separate the non-single stranded code regions. In **FIG. 1****,** a single-stranded segment **110** with a detectable property and positioned between code region **107** and code region **108** can serve as another code region. In some embodiments, the single stranded code region can be a block polymer, block copolymer, or alternating copolymers as described above. Sensing the detectable properties of the plurality of single stranded and non-single stranded code regions (*e.g.,* **105, 106,** and **107**) generates a reproducible signal pattern that defines the coded molecule and which allows the molecule to be distinguished from coded molecules with a different set of code regions. A single-stranded probe region **109** serves as a moiety for binding to a specific target sequence **112.**

For purposes of stabilizing the hybridized nucleobase oligomers that form part of a code region, the strands can be crosslinked **111** by any suitable crosslinking agent, as elaborated below. By crosslinking the strands of the non-single-stranded code region, the coded molecule is stable and can be subjected to conditions that would otherwise destabilize the code region, for example high voltage gradients capable of denaturing the nucleobase oligomer when translocating the coded molecule through a nanopore. In addition, a stable code region can minimize variations in the structural conformations of the code region.

As will be apparent to the skilled artisan, other coded molecules displaying a different signal pattern can be formed by changing the order of the nucleobase oligomers hybridized to the single-stranded nucleobase polymer scaffold. This is illustrated in **FIG. 2** in which the sequence of the scaffold is altered to switch the hybridization positions of the nucleobase oligomers **102** and **103** shown in **FIG 1****.** **FIG 2** shows two types of coded molecules. A first coded molecule **200** has code region **201, 202,** and **203** formed by positioning sequentially on the first scaffold nucleobase oligomers **101, 102,** and **103.** A second coded molecule **210** is formed by positioning sequentially on the second scaffold the nucleobase oligomers **101, 103,** and **102.** Thus, the order of code regions **202** and **203** are switched between coded molecules **200** and **210.** Since a coded molecule is scanned sequentially during translocation through the nanopore, the change in order of the code region generates two different signal patterns if code regions **202** and **203** are distinguishable from each other. Differences in the detectable properties of code region **202** from code region **203** can be created by sequence differences, by use of chemically different nucleobase oligomers **102** and **103** (*e.g.,* PNA and charged PNA), or other methods (*e.g.*, detectable tags) as described herein. A first probe sequence **220** present on the first coded molecule **200** serves as a first moiety for binding to a first target sequence **221,** while a second probe sequence **222** present on the second coded molecule **210** serves as a second moiety for binding to a second target sequence **223.** Where the probe sequences **220** and **222** have different sequences, different target sequences are detectable. In a mixture of coded molecules, coded molecules of the structure **200** form a first subpopulation and coded molecules of the structure **210** form a second subpopulation, where the population of coded molecules is capable of detecting two different target analytes.

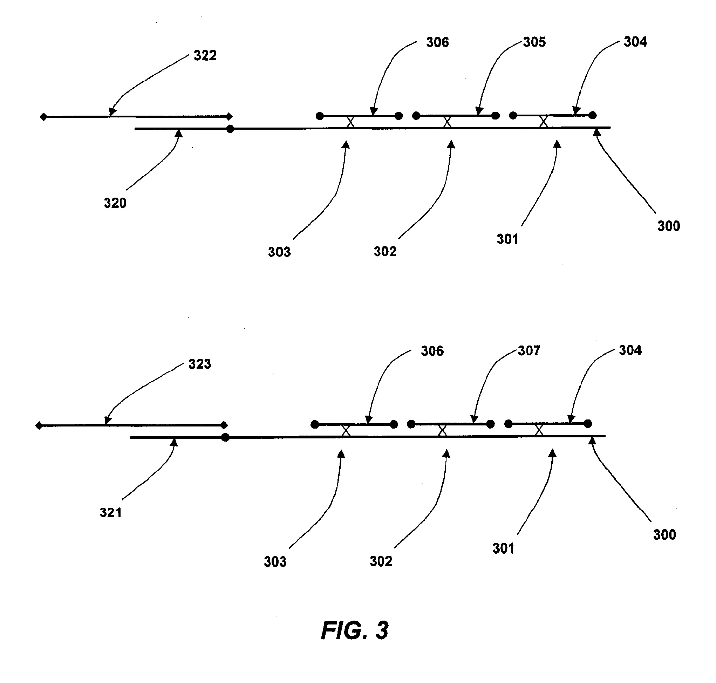

In some embodiments, different coded molecules can be generated on identical scaffolds by using combinations of nucleobase oligomers in which each combination of nucleobase oligomers hybridizes to the same set of sequences on the single-stranded scaffold. However, one or more of the nucleobase oligomers between each combination differ as to the type of nucleobase polymer (*e.g.,* PNA, DNA, or RNA). These embodiments are illustrated in **FIG. 3****,** which shows two types of coded molecules. A first coded molecule is generated from a single stranded scaffold **300** having code regions **301, 302,** and **303** formed by positioning sequentially on the first scaffold nucleobase oligomers **304, 305,** and **306.** Another coded molecule is generated from a scaffold **300** identical to that used in the first coded molecule. Positioned sequentially on the second scaffold are the nucleobase oligomers **304, 307,** and **306.** The nucleobase polymers **305** and **307** hybridize to the identical sequence on the scaffold but are made of different types of nucleobase polymers (*e.g.*, DNA versus PNA). The difference in the type of nucleobase polymer hybridized to code region **302** provides the basis to distinguish the coded molecules. A first probe sequence **320** present on the first coded molecule serves as a first moiety for binding to a first target sequence **322,** while a second probe sequence **321** present on the second coded molecule serves as a second moiety for binding to a second target sequence **323.** It is to be understood that other coded molecules can be generated by employing different types of nucleobase polymers in the other non-singled-stranded code regions.

In accordance with the above, in some embodiments, a method of making different coded molecules can comprise contacting a first combination of nucleobase oligomers with a first single-stranded scaffold and contacting a second combination of nucleobase oligomers with a second singled stranded scaffold, wherein the first and second scaffold molecules are identical and the first and second combination of nucleobase oligomers are capable of hybridizing to the same defined code regions on the scaffolds. To generate different coded molecules on identical scaffolds, at least one of the nucleobase oligomers between the first and second combination of nucleobase oligomers differ as to the type of nucleobase polymer. The nucleobase oligomers of each combination can be hybridized sequentially, or hybridized in combination, either whole or in part, to the single-stranded scaffold. Use of reaction compartments to sequester a scaffold and nucleobase oligomers to generate different coded molecules are further described below.

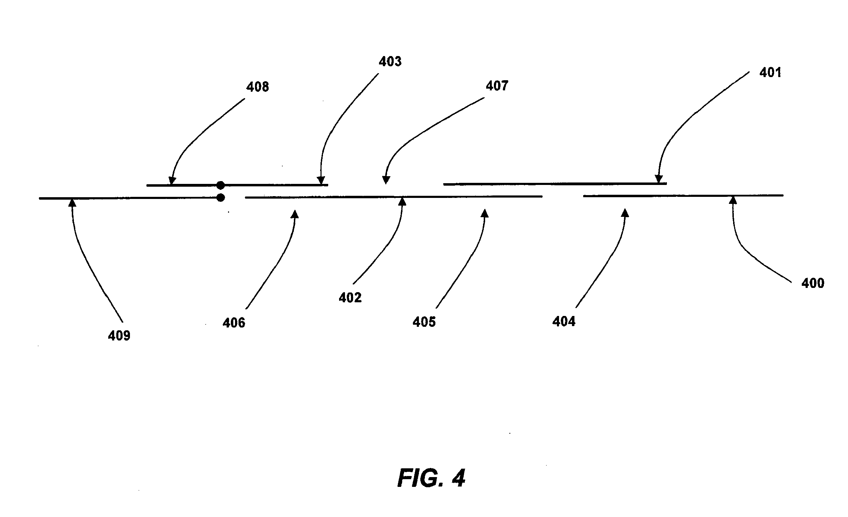

A coded molecule generated from segments of single-stranded nucleobase polymers is shown in **FIG. 4****.** Four single-stranded nucleobase polymers **400, 401, 402,** and **403** are designed to have overlapping complementary segments at the terminal regions of the polymers such that they hybridize to each other to form a single linear molecule. A single-stranded region **407** can separate the non-single-stranded regions and optionally operate as a code region. In the illustrated coded molecule, the hybridized regions operate as code regions **404, 405,** and **406.** A single-stranded probe region **408** on nucleic acid segment **403** can serve as a moiety for binding to a specific target sequence. The single-stranded segments **400, 401, 402** and **403** can be the same type of nucleobase polymer (e.g., DNA) or be composed of different nucleobase polymers. For instance, if segment **400** is a PNA and the segment **401** is a DNA, the code region **404** is a DNA-PNA hybrid. By having the segment **402** as a DNA, code region **404** and **405** can be distinguished not only by their sequence differences but also by differences in nucleic acid structure.

Other variations of the coded molecules will be apparent to the skilled artisan from the descriptions and embodiments presented herein.

### 5.2.1 Detectable Tags

Each code region of a coded molecule has an associated detectable property that identifies its presence on the coded molecule. In some embodiments, the code region further comprises a detectable tag capable of producing a signal upon interrogation of the code region. A "detectable tag" refers to any detectable atom, molecule, compound or composition. The detectable tag can be attached, either covalently or noncovalently, to the scaffold and/or one or more nucleobase polymer strands comprising the non-single-stranded code regions. Various detectable molecules are known in the art, and include, among others, fluorescent, phosphorescent, luminescent, electroluminescent, and electron transfer compounds. Additional detectable labels include Raman labels, nanoparticles, quantum dots, and sterically bulky groups.

In some embodiments, the detectable tag comprises an optical label, such as a dye or fluorescent, phosphorescent, electroluminescent compound, or a compound that affects the response of an optical tag. Fluorophores suitable for incorporation into nucleobase polymers are described in Molecular Probes Handbook, 10th Ed., R.P Haugland ed., Molecular Probes, Eugene, OR (2005); Smith et al., 1987, Meth Enzymol 155:260-301, Karger et al., 1991, Nucl Acids Res 19:4955-4962. Suitable fluorescent molecules include flurophores based on xanthene, fluorescein (such as disclosed in U.S. Patent Nos. 4,318,846 and 6,316,230, and Lee et al., 1989, Cytometry 10:151-164), rhodamine, cyanine, phthalocyanine, squaraine, and bodipy dyes. Exemplary fluorescent dyes include, by way of example and not limitation, 6-FAM, JOE, TAMA, ROX, HEX-1, HEX-2, ZOE, TET-1, NAN-2, 5- and 6-carboxyfluorescein, 5- and 6-carboxy-4,7-dichlorofluorscein, 2',7'-dimethoxy-5- and 6-carboxy-4,7-dichlorofluorescein, 2',7'-dimethoxy-4',5'-dichloro-5- and 6-carboxyfluorescein, 2',7'-dimethoxy-4',5'-dichloro-5- and 6-carboxy-4,7-dichlorofluorescein, 1',2',7',8'-dibenzo-5- and 6-carboxy-4,7-dichlorofluorescein, 1',2',7',8'-dibenzo-4',5'-dichloro-5- and 6-carboxy-4,7-dichlorofluorescein, 2',7'-dichloro-5- and 6-carboxy-4,7-dichlorofluorescein, and 2',4',5',7'-tetrachloro-5- and 6-carboxy-4,7-dichlorofluorescein (see, *e*.*g*., U.S. Patent Nos. 4,997,928; 4,855,225; and 5,188,934).

In other embodiments, the detectable tag is an electron transfer label. An "electron transfer label" refers to a compound that facilitates transfer of electrons from an electron donor to an electron acceptor molecule. Thus, the electron transfer compound can be itself an electron donor/acceptor molecule. Exemplary electron transfer labels are organometallic compounds, including, by way of example and not limitation, metallocenes (*e.g.,* ferrocene), phenanthrolines (*e.g.,* 1,10-phenanthroline), bipyridines (*e.g.,* 2,2'-bipyridine), tripyridines (*e.g.,* 2,2',2" terpyridine), and porphyrin macrocyles (*e.g.,* porphyrin (meso-tetrakis(N-methyl-x-pyridinium)porphyrin tetrachloride) complexed to various transition metals, such as Cr, Fe, Pt, and Ru. As will be apparent to those skilled in the art, some of the electron transfer labels can also have fluorescent properties, for example, 2,2'-bipyridine and 1,10-phenanthroline complexes. Various other compounds capable of acting as electron acceptor/donor molecules include, among others, riboflavin, xanthene dyes, azine dyes, acridine orange, quinones such as methylene blue, Nile blue A (aminoaphthodiethylaminophenoxazine sulfate), anthracene, coronene, pyrene, 9-phenylanthracene, rubrene, binaphthyl, phenothiazene, fluoranthene, chrysene, naphthalene, acenaphthalene, perylene, and analogs and substituted derivatives of these compounds. Attachment of electron transfer labels to nucleobases polymers are described in U.S. Patent Nos. 6,096,273 and 6,221,583.

In other embodiments, the detectable tag is a nanoparticle. As used herein, a "nanoparticle" refers to a particle that is of nanometers in size and has a detectable property (*e.g.,* electrical, optical, magnetic, steric, etc.). Typical sizes ranges of nanoparticles are about 1 to about 3 nm, but nanoparticles of smaller (less than 1 nm in diameter) or larger dimensions (*e.g.,* about 30 nm in diameter) can be used. Exemplary nanoparticles include gold and silver nanocrystals formulated as colloids, which can be attached to nucleic acids and proteins with a thiol (-SH) functional group (see, *e.g.,* U.S. Patent No. 6,054,495; 6,127,120; and 6,149,868). Other forms of nanoparticles can be prepared from semiconductor material as further described below. Nanoparticles derivatized with other functional groups, including maleimide and amino groups, are also available for attachment to biological molecules. Detection of the nanoparticle exploits any detectable property exhibited by the nanoparticles. Surface plasmon resonance detection of single nanoparticles is described in Nichtl et al., Nano Lett. 3(7):935-938 and Schultz et al., 2000, Proc Natl Acad Sci USA 97(3): 996-1001. The magnitude, peak wavelength, and spectral bandwidth of the plasmon resonance associated with a nanoparticle are dependent on the particle's size, shape, and material composition, as well as the local environment. By manipulating these parameters during preparation, nanoparticles having differing plasmon resonance properties can be made. Single nanoparticles can also be detected by their optical and light scattering properties using techniques such a near field scanning optical, total internal reflection, and dark field microscopy.

In other embodiments, the nanoparticles are quantum dots, which are nanocrystals typically made of semiconducting materials, having dimensions between a nanometer to a few microns in diameter. In a typical quantum dot, electrons are confined in all three dimensions, which modify their interactions and their energy spectrum to the extent that they behave analogously to atoms where electrons occupy well-defined, discrete quantum states. The size and shape of these structures, and therefore the number of electrons they contain, can be precisely controlled. A quantum dot can have from a single electron to a collection of several thousand electrons. Quantum dots can be made from various materials, including but not limited to, the Group II-VI, III-V and IV semiconductors. Exemplary materials suitable for use as quantum dots include ZnS, ZnSe, ZnTe, CdS, CdSe, CdTe, GaN, GaP, GaAs, GaSb, InP, InAs, InSb, AIS, AlP, AlAs, AlSb, PbS, PbSe, Ge, and Si and ternary and quaternary mixtures thereof (see, *e.g.,* U.S. Patent No. 6,872,249; U.S. Published Application No. 2001/0040232). In some embodiments, the quantum dots can be made of different layers of materials. These include as examples a quantum dot with an inner core made of a first semiconductor material and an outer layer of a different semiconductor material or a coating of hydrophilic layer (Lin et al., 2005, Langmuir 21(2):728-34; Medintz, 2005, Nature Mater 44(6):435-46). An exemplary quantum dot of this type comprises an inner core of CdSe and and outer layer of ZnS (Dabbousi et al., 1997, J Phys Chem B 101:9463-6475). Covalent bonding, ionic bonding, and/or hydrophobic interactions can be used to generate the outer layer on the semiconductor surface.

In some embodiments, various labels and compounds can be attached to the quantum dot via functional groups on the quantum dot surface (Medintz et al., *supra*). Suitable functional groups include, among others, amines, thiols, carboxyls, phosphines, amine oxides, and phosphine oxides. A linking moiety with a reactive functional group can be used to attach the compounds to the quantum dot surface, and thereby provides a method of attaching quantum dots to nucleobase polymers (U.S. Patent No. 6,582,921; Medintz, *supra*). Methods for making quantum dots are described in various publications (U.S. Published Applications 2004/0259363 and 2001/0040232; U.S. Patent Nos: 6,918,946, 6,861,155, 6,846,674, 6,846,565, 6,680,211, 6,582,921, 6,548,264, 5,690,807, and 5,474,591).

In still other embodiments, the detectable tag is a steric modifier that alters the structure or conformation of the code region such that the code region displays a detectable property distinguishable from the code region without the steric modifier. A steric modifier encompasses electron transfer moieties as well as optical molecules that change the structure sufficiently to alter the property detected based on steric characteristics, such as current blockade or electron tunneling current described below. Other modifiers include compounds that conjugate to nucleic acids and cause changes in nucleic acid structure, sometimes referred to as bulky adducts. A general class of bulky adduct compounds is activated polycyclic aromatic hydrocarbons. Activation generally involves epoxidation of the aromatic hydrocarbon that ultimately leads to formation of a reactive diol epoxide. Non-limiting examples of aromatic hydrocarbons include, among others, activated forms of benzophenanthracene and benz(a)pyrene. Another class of steric modifiers is aromatic amines, which typically modify the C8 position of guanine. Aromatic amines also require activation by cellular enzymes. Non-limiting examples of aromatic amines that form adducts with nucleic acids include, among others, aminofluorine, acetylaminofluorine, 3-amino-1-methyl-5H-pyrido[4,3-b]indole, 2-amino-6-methyldipyrido[1,2-a:39, 29-d]imidazole, and 2-amino-3-methyl-imidazo-[4,5-f]quinoline. Additional embodiments of steric modifiers are N-nitrosoamines and N-nitrosoureas capable of forming small alkyl adducts, which, though not considered bulky adducts, alters the structure of nuclei acids. Non-limiting examples of alkylating agents of this type include, among others, N-nitrosodimethylamine, 4-(methylnitrosoamino)-1-(3-pyridyl)-1-butanone, and N,N'-bis(2-chloroethyl)-N-nitrosourea (*e.g.*, Pieper et al., 1995, Mol Pharmacol 47(2):290-5).

Some steric modifiers are activated forms of metabolites generated by microorganisms. Alfatoxins (*e.g.*, aflatoxin B₁, B₂, G₁, and G₂) are products of fungus *Aspergillus flavus,* and activation of aflatoxin B₁ and G2 by cytochrome P450 enzymes generates compounds that form covalent adducts with DNA. Other bulky adduct generating agents, include, among others, cis-platin (*e*.*g*., Poklar et al., 1996, Proc Natl Acad Sci USA 93(15):7606-11) and epoxides (*e*.*g*., butadiene epoxide; Carmical et al., 2000, J. Biol. Chem. 275(26) 19482-19489). Other bulky adducts will be apparent to the skilled artisan (e.g., Kowalczyk et al., 2000, Biochemistry 41:3109-3118; Gibbs et al., J Photochem Photobiol B. 2(1):109-22).

The detectable tag can be present on one or more of the code regions. Different combination of detectable tags can be used to generate a large number of code combinations that permit distinguishing one coded molecule from another coded molecule. Placement of the detectable tag can vary in each code region. In some embodiments, the detectable tag resides on the defined positions on the scaffold, where defined position serves as a code region. In other embodiments, the detectable tag is not on the scaffold, but is present on the non-scaffold strands of the multistranded region. Each code region can have one detectable tag or have a plurality of detectable tags, in which case the detectable tags can be the same or different. Where the same detectable tags are present, signal strength could be the signal distinguishing a code region from a code region having a fewer number of the same detectable tags. Where different detectable tags are present, each tag can be interrogated depending on the detectable property of the tag. Thus, in some embodiments, the coded molecule can be scanned using different detectors.

Detectable tags can be attached to the code region covalently or by non-covalent processes. Thus, in some embodiments, the detectable tag can be attached by intercalation into a multistranded code region. Intercalation generally does not affect the Watson and Crick base pairing of a double-stranded nucleic acid although there is short range and long range disruptions to a double-stranded nucleic acid structure because the double stranded nucleic acid must accommodate the insertion of the compound into the stacked nucleobases. Intercalation generally causes a stiffening of the nucleic acid duplex, unwinding of the strands, and lengthening of the helix. Because there is some sequence specificity to the binding of intercalating compounds, code regions with differing nucleic acid sequences can display different binding capacities, and thereby display different properties from other code regions. Non-limiting examples of intercalating compounds, many of which are also fluorescent, include, among others, aminoacridine, acridine orange, proflavin, ethidium, ellipticine, 3,5,6,8-tetramethyl-N-methylphenanthrolinium, 2-hydroxy-ethanethiolateo-2,2',2"-terpyridine-platinum (II), daunomycin, and actinomycin. Intercalating compounds are described in various references, such as Molecular Probes Handbook, 10th Ed., R.P Haugland ed., Molecular Probes, Eugene, OR (2005).

### 5.2.2 Binding Moiety

For the detection of target analytes, the coded molecules further comprise a binding moiety such that the binding moiety and the target analyte form a binding pair. The binding moiety specifically binds to the target analyte. "Specific binding" is binding that is determinative of the presence of a target analyte in the presence of a heterogeneous population of analytes. A "non-specific binding" is an affinity of a binding moiety for one or more targets as well as non-target molecules. Thus, under a designated assay condition, the specific binding moieties bind preferentially to a particular target analyte and discriminates the target analyte from the other non-target analytes present in a test sample. Generally, a specific or selective reaction will be at least twice non-specific binding (*e*.*g*., background binding), typically have a dissociation constant (K_{D}) of at least about 10⁻² M, at least about 10⁻³ M, at least about 10⁻⁶ M, at least about 10⁻⁷ M, at least about 10⁻⁸ M, at least about 10⁻⁹ M, at least about 10⁻¹⁰ M, at least about 10⁻¹² M, or at least about 10⁻¹⁵ M, or higher.

In some embodiments, the binding moiety is a polynucleotide probe that binds a target polynucleotide sequence. As discussed below, the probe has a sequence that is complementary to a sequence on the target nucleic acid and forms a stable hybridization complex with the target nucleic acid. The length of the probe can be any length sufficient to specifically hybridize to the target sequence. Thus, the polynucleotide probe can be 8 or more nucleobases, 10 or more nucleobases, 20 or more nucleobases, 50 or more nucleobases, 100 or more nucleobases in length up to thousands of nucleobases. Naturally occurring polynucleotides or synthetic polynucleotide analogs can be used to form the probe sequence.

In various embodiments, the probe sequence can be complementary to a sequence of genomic DNA (gDNA); RNA (e.g., mRNA; noncoding RNA, tRNA, siRNA, snRNA; mitochondria or chloroplast DNA; nucleic acid obtained from microorganisms (*e.g.*, fungi, bacteria); parasites (*e.g.,* trypanosomes, nematodes, helminthes); DNA or RNA viruses; and synthetic nucleobase sequences (e.g., sequences for isolating a PCR product). In some embodiments, the probe sequence is directed to a pathogenic organism for detecting presence of the pathogen. Non-limiting examples of pathogenic organisms include, among others, *Salmonella, Campylobacter, Vibrio cholerae, Leishmania,* enteric *E. coli,* retroviruses, herpesviruses, adenoviruses, and lentiviruses. In still other embodiments, the polynucleotide probe sequences are directed to variants of a specific pathogen. For instance, drug resistance human immunodeficiency virus can arise from mutations genes that encode the molecule targeted by an anti-retroviral drug. For instance, mutations in HIV protease enzyme are known to produce resistance to protease inhibitors used for HIV therapy. Thus, the polynucleotide probe sequences that distinguish the various mutations can be used in the coded molecules.

In some embodiments, the polynucleotide probe sequences are complementary to mutated sequences responsible for hereditary disorders. Non-limiting examples include, among others, mutations responsible for ataxia telangiectasia, canavan disease, cystic fibrosis, Fanconi anemia, Gaucher disease, hereditary nonpolyposis colorectal cancer, hemophilia, Huntington's disease, leukodystrophy, neurofibromatosis, osteogenesis imperfecta, porphyria, retinoblastoma, sickle cell disease, Tay-Sachs, Treacher Collins syndrome, and tuberous sclerosis. Various mutations causing each genetic disorder can be detected by use of a pair of coded molecules for each mutation site, where one coded molecule has a polynucleotide probe for the normal sequence and another coded molecule has a polynucleotide for the mutated sequence. Such pairs of coded molecules and associated polynucleotide probes can be made for each mutation known to cause a genetic disorder.

In still other embodiments, the polynucleotide probe sequences are complementary to single nucleotide polymorphisms (SNP) present in a population of subjects. Detection of SNPs is valuable for a number of reasons. First, SNPs can provide information about risk factors for certain diseases. For instance, SNP variation in the apolipoprotein E (ApoE) is correlated with an increased risk for Alzheimer's Disease. The ApoE gene displays polymorphisms predominantly at two nucleotide positions that result in three possible alleles for this gene: ε2, ε3, and ε4. Each allele, differing by one base, produces a protein product that differs by one or two amino acids from the other alleles. An individual inheriting at least one ε4 allele has an increased risk of developing Alzheimer's while inheriting the ε2 allele is not associated with an increased risk. Second, SNPs are useful in determining relationships of subjects in the fields of forensics, paternity testing, and evolutionary studies. Knowledge of the frequency at which a combination of SNPs occurs in a population provides high discrimination in determining the relationship of one individual to another individual. Third, screening for SNPs when administering pharmacological agents can provide a basis for assessing the response of the individual to the drug, such as adverse reaction or drug efficacy. For example, differences in the toxicity and/or efficacy profile of some drugs arise in part from metabolism by cytochrome P450 enzymes, a superfamily of heme containing monooxygenases. Human cytochrome P450 enzyme families, such as CYP1, CYP2, and CYP3, metabolize various drugs and environmental chemicals such that differences in the activities of specific enzymes within each cytochrome P450 family can affect drug metabolism (Gonzalez, F.J., 1992, Trends Pharmacol Sci 13(9):346-52). For instance, an SNP that results in low or no expression of CYP2C9 can increase the risk of adverse effects of taking tolbutamide or coumadin because of the low metabolism of these drugs in subjects carrying the SNP in the CYP2C9 (Schwarz, U.I., 2003, Eur J Clin Invest 33 Suppl 2:23-30). Thus, detecting polymorphisms in these and other drug metabolizing enzymes (*e.g.*, esterases) can be used in predicting response to a drug.

In other embodiments, the binding moiety is a protein or peptide that binds specifically to a target analyte. Non-limiting examples include, among others, antibodies (natural or synthetic, monoclonal or polyclonal, humanized, single chain, etc.), receptors or the cognate peptide ligands (*e*.*g*., peptide hormones, cytokines, etc.), protein interacting domains, nucleic acid binding proteins, metal binding proteins, lipid binding proteins, and sugar binding proteins. Any such protein or peptide can be attached to the coded molecule and then processed, as further described below, to detect the presence of their cognate target analytes. Non-limiting examples of protein binding domains include, among others, SH2 domain (src homology domain 2), SH3 domain (src homology domain 3), PTB domain (phosphotyrosine binding domain), FHA domain (forkedhead associated domain), WW domain, 14-3-3 domain, pleckstrin homology domain, Cl domain, C2 domain, FYVE domain (Fab-1, YGL023, Vps27, and EEA1), death domain, death effector domain, caspase recruitment domain, Bcl-2 homology domain, bromo domain, F box domain, hect domain, PDZ domain (PSD-95, discs large, and zona occludens domain), ankyrin domain, arm domain (armadillo repeat motif), WD 40 domain and EF-hand (calretinin), and PUB domain (Suzuki T. et al., 2001, Biochem. Biophys. Res. Commun. 287:1083-87). In other embodiments, the binding moiety comprises a candidate binding moiety that can be used to identify binding moieties specific to a target analyte (see, *e.g.,* U.S. Published Application No. 2004/0248109). The protein or peptide based binding moieties can be used in isolation or used with protein scaffolds for the display of the binding moieties to enhance binding interactions. Exemplary protein scaffolds include, among others, minimal antibody complementarity region, coiled-coil structures, β-loop structure, zinc-finger domains, and cysteine-linked (disulfide) structures (see, *e.g.,* U.S. Patent No. 6,455,247; Nygren and Skerra, 2004, J Immunol Meth 290:3-28).

In other embodiments, the binding moiety is a small molecule ligand that binds the target analyte. As used herein, a small molecule is generally an organic molecule of about 200 to about 10,000 daltons. Exemplary small molecules useful for detecting target analytes include, among others, biotin, haptens, steroids, lipids, therapeutic molecules (*e.g.*, chemotherapeutic compounds, antibiotics, opiates, etc.), enzyme inhibitors, carcinogens, macrocycles (*e.g.*, porphyrins), and chelators. Other small molecule binding moieties will be apparent to the skilled artisan.

In various embodiments, the binding moiety is attached directly to the coded molecules, for instance the scaffold, while in other embodiments the binding moiety is attached indirectly via a linker. Where the binding moiety is a polynucleotide sequence, it can be contiguous with the scaffold in that the scaffold comprises sequences that hybridize to a target sequence. In other embodiments, the target sequence can be attached through another polynucleotide segment, such as a capture probe comprising a first region that hybridizes to a region on the coded molecule scaffold and a second region that binds the target nucleic acid. The first region can have a sequence that hybridizes to a common sequence on all coded molecules or comprise different sequences that hybridize to different coded molecules. In some embodiments, where the coded molecule comprises nucleobase polymer segments hybridized to each other (*e.g.*, **FIG. 3**), one of the segments can comprise the capture probe.

In some embodiments, the binding moiety is attached to the scaffold via a linker. As used herein, a "linker" refers to a chemical moiety comprising a covalent bond or a chain of atoms that covalently attaches the binding moiety to the coded molecule. Linker molecules are available with varying lengths of spacer arms or bridges, which typically connect two reactive groups on the linker molecule used for attachment. The linker can be used during synthesis of the coded molecule or used subsequent to synthesis. Various types of linkers can be used. In some embodiments, the linkers can be crosslinking agents that react with a functional group on the binding moiety and the coded molecule. Crosslinking agents can be homobifunctional or heterofunctional. Homobifunctional agents have the same reactive molecules in the linker while heterofunctional crosslinkers have different reactive molecules in the linker. Reactive groups on the homofunctional and heterofunctional crosslinkers include, among others, imidoesters, N-hydroxysuccinimide esters, maleimides, haloacetyls, pyridyldisulfides, hydrazides, and carbodiimides. Crosslinking agent can also be based on a photoreactive group, such as arylazides. These and other crosslinking agents are described in various references works and publications, such as Hermanson, G.T., 1996, Bioconjugate Techniques, Academic Press, Inc., San Diego, CA; Pierce Applications HandbooklCatalog, 2005, Pierce Chemicals; Double-Agents Cross-Linking Guide, 2003, Pierce Biotechnology, Publication No. 1600918; U.S. Patent No. 6,320,041; Morocho et al., 2004, Bioconjugate Chem 15:569-575*;* Shchepinov et al., 1997, Nucleic Acids Res 25(6):1155-1161; and Shea et al., 1990, Nucleic Acids Res 18(13):3777-3783. For use in attaching a binding moiety during synthesis of the coded molecules, various linkers with protecting groups can be used.

In other embodiments, the linker is a cleavable linker that, when subjected to the appropriate conditions, cleaves to separate the binding moiety from the scaffold. The cleavable linker may be cleavable by a chemical agent, an enzyme, or by photoreaction (see, *e.g.,* Lloyd-Williams et al., 1993, Tetrahedron 49, 11065-11133). Non-limiting examples of chemically cleavable linkers include, among others, vinyl sulphones (WO 00/02895); base-cleavable sites, such as esters (e.g., succinates) cleavable using, for example, ammonia or trimethylamine; acid-cleavable sites, such as benzyl alcohol derivatives, cleavable using trifluoroacetic acid, acetals and thioacetals; dithiols cleavable by thiol compounds (see, *e.g*., Thevenin et al., 1992, Eur. J. Biochem. 206(2):471-7); sulfonyl compounds cleavable by trifluoromethane sulfonic acid, trifluoroacetic acid, thioanisole; diisopropyldialkoxysilyl linkers cleavable by fluoride ions; and hydrazone linkers (Laguzza et al, 1989, J. Med. Chem. 32:548-555). Other cleavable linkers are described in the literature, for example, Brown, 1997, Contemporary Organic Synthesis 4(3):216-237; W. A. Blattler et al, Biochemistry 24:1517-1524 (1985); Wong, S.S., 1993, Chemistry of Protein Conjugation and Cross-linking, CRC Press, BocaRaton, FL.; and U.S. Pat. Nos. 4,542, 225, 4,569,789, 4,618,492, and 4,764, 368.

Enzymatically cleavable linkers can use peptides with sequences recognized by specific proteases or polynucleotide sequences recognized by sequence specific nucleases. Classes of proteases useful as a cleaving agent include, among others, serine proteases; cysteine proteases, aspartic proteases, and metallo-proteases. Non-limiting examples of proteases include chymotrypsin, trypsin, elastase, subtilisin, bromelain, papain, cathepsins, pepsin, renin, thermolysin, and various fungal and viral proteases (see, e.g., Handbook of Proteolytic Enzymes, 2003, 2nd Ed., A. Barrett, N. Rawlings, and F. Woessner, eds., Elsevier Science, Oxford, UK. In other embodiments, where the linker is a polynucleotide, sequence specific nucleases can be used as the cleavage agent. Restriction endonucleases, for instance Type II restriction endonucleases, can be used for such purposes (see, *e.g.,* Williams, R.J., 2003, Mol Biotechnol. 23(3):225-43; New England Biolabs Catalog, 2005, New England Biolabs, Ipswich, MA).

In other embodiments, use of photolabile linkers for the attachment of molecules provides a method of separating the binding moiety from the coded molecule under mild reaction conditions. Various photolabile linkers are known in the art (see, *e.g.,* Lloyd-Williams et al., 1993, Tetrahedron 49:11065-11133; Gallop et al., 1994, J Med Chem 37:1233-1251; and Gordon et al., 1994, J. Med. Chem. 37:1385-1401; Ottl et al., Bioconjugate Chem 9(2):143-151). Exemplary photocleavable linkers can be based on ortho-nitrobenzyl groups, including 2-nitrobenzyl esters and 2-nitrobenzylamines (Senter et al, 1985, Photochem Photobiol 42:231-237; Holmes, C., 1997, J Org Chem 62:2370-2380; Holms and Jones, 1995, J Org. Chem 60:2318-2319; Akerblom, E., 1999, Molecular Diversity 4:53-69). Other examples of photolabile linker include, among others, 4,4-bis(alkoxymethyl-3,3-dinitro)biphenyl, as described in Madhavan et al., 2004, Chem Comm 23:2728-2729 and 3', 5'-dialkoxybenzoin, as described in Cano et al., 2002, J Org Chem 67:129-135.

As will be apparent to the skilled artisan, other variations of the coded molecules are contemplated. For example, a capture probe that hybridizes to the scaffold and comprises a region that binds to a target analyte can be used. In other embodiments, the coded molecule can have a bound target sequence and a detection probe that binds to the target probe but not to the scaffold to detect the target analyte in a "sandwich" format.

### 5.2.3 Synthesis of Coded Molecules

The nucleobase polymer scaffold and the nucleobase polymer for generating non-single-stranded code regions can be made by standard methodologies known in the art. The single-stranded scaffold can be synthesized in whole or in parts, where the parts are subsequently joined together. The resultant polymeric coded molecule can then be conjugated to the binding moiety.

Recombinant techniques can also be used to synthesize the coded molecule, or part thereof (see, *e.g.,* Sambrook et al., *supra;* Ausuble et al., *supra*). For instance, single-stranded polynucleotides are readily made using single-stranded phage systems, as described above. Cloned fragments of naturally occurring sequences as well as amplification products can also be used for constructing the coded regions. In other embodiments, nucleobase polymers can be synthesized using standard chemistries (see, *e.g.,* Current Protocols in Nucleic Acid Chemistry, John Wiley & Sons, 2003; U.S. Patent No. 4,973,679; Beaucage, 1992, Tetrahedron 48:2223-2311; U.S. Patent No. 4,415,732; U.S. Patent No. 4,458,066; U.S. Patent No. 5,047,524 and U.S. Patent No. 5,262,530). The synthesis can be accomplished using automated synthesizers available commercially, for example the Model 392, 394, 3948 and/or 3900 DNA/RNA synthesizers available from Applied Biosystems, Foster City, CA.

Methods for synthesizing nucleobase oligomer and polynucleotide analogs and mimetics will follow standard methodologies. For example, PNAs are described in U.S. Patent Nos. 5,539,082; 5,527,675; 5,623,049; 5,714,331; 5,718,262; 5,736,336; 5,773,571; 5,766,855; 5,786,461; 5,837,459; 5,891,625; 5,972,610; 5,986,053; 6,107,470; 6,201,103; 6,350,853; 6,357,163; 6,395,474; 6,414,112; 6,441,130; and 6,451,968. General descriptions for PNA synthesis methodologies are given in Nielsen et al., 1999, Peptide Nucleic Acids; Protocols and Applications, Horizon Scientific Press, Norfolk England.

Where the scaffold is a composite of non-polynucleotide and polynucleotide polymers, the scaffold can be synthesized in segments and then assembled together or, alternatively, formed by sequential synthesis of the non-polynucleotide polymer region and the polynucleotide polymer region. For example, phosphoramidite polyethylene glycols along with phosphoramidite nucleotides for synthesis of nucleic acid-PEG composite scaffolds (Sanchez-Quesada et al., *supra*) can be used as precursors for synthesizing a composite polymer of polynucleotides and PEG.

For synthesis of the non-single-stranded code regions, various methodologies can be used. In some embodiments, a nucleobase polymer is hybridized to a defined region of the single-stranded scaffold. The nucleobase polymer should be of sufficient length such that a stable hybridization complex with the scaffold is formed and, where appropriate, of sufficient length to provide a detectable property for the code region. A detectable property, such as current blockade or electron tunneling current, for a non-single-stranded code region can be determined by translocating the coded molecule through a nanopore and scanning the coded molecule using the desired detection technique. Hybridization of the nucleobase polymer to the scaffold can depend on a number of factors, including, but not limited to, the thermal melting temperature of the nucleobase polymer and the scaffold sequence, salt concentration, ionic strength, pH, and other buffer conditions. In various embodiments, a code region can be from about 5 nucleobases or longer, about 8 nucleobases or longer, about 15 nucleobases or longer, about 50 nucleobases or longer, about 100 nucleobases or longer, about 500 nucleobases or longer, about 1000 nucleobases or longer, up to about 5,000 nucleobases. It is to be understood that the length of the code region can be longer where suitable. The ability to select lengths and sequences of suitable for a particular application is within the capabilities of a person skilled in the art (see, *e.g.,* Sambrook et al. *supra*; Ausuble et al., *supra*).

Annealing characteristics of a nucleobase polymer can be determined by the *Tₘ* of the hybrid complex. The greater the *Tₘ* value, the more stable the hybrid. *Tₘ* is the temperature at which 50% of a nucleobase oligomer and its perfect complement form a double-stranded oligomer structure. The *Tₘ* for a selected nucleobase polymer also varies with factors that influence or affect hybridization. For example, such factors include, but are not limited to, factors commonly used to impose or control stringency of hybridization, (*i.e.,* formamide concentration (or other chemical denaturant reagent), salt concentration (*i.e.,* ionic strength), hybridization temperature, detergent concentration, pH and the presence or absence of chaotropes. Optimal stringency for forming a hybrid combination can be found by the well-known technique of fixing several of the aforementioned stringency factors and then determining the effect of varying a single stringency factor. The same stringency factors can be modulated to control the stringency of hybridization of a PNA to a scaffold, except that the hybridization of a PNA is fairly independent of ionic strength. Optimal or suitable stringency for an assay can be experimentally determined by examination of each stringency factor until the desired degree of discrimination is achieved.

The *Tₘ* values for the nucleobase oligomers can be calculated using known methods for predicting melting temperatures (see, *e.g.,* Baldino et al., Methods Enzymology 168:761-777; Bolton et al., 1962, Proc. Natl. Acad. Sci. USA 48:1390; Bresslauer et al., 1986, Proc. Natl. Acad. Sci USA 83:8893-8897; Freier et al., 1986, Proc. Natl. Acad. Sci USA 83:9373-9377; Kierzek et al., Biochemistry 25:7840-7846; Rychlik et al., 1990, Nucleic Acids Res 18:6409-6412 (erratum, 1991, Nucleic Acids Res 19:698); Sambrook et al., *supra*); Suggs et al., 1981, In Developmental Biology Using Purified Genes (Brown et al., eds.), pp. 683-693, Academic Press; and Wetmur, 1991, Crit Rev Biochem Mol Biol 26:227-259.

In some embodiments, a nucleobase oligomer is substantially complementary to a defined segment of the single-stranded scaffold. By "substantially complementary" herein is meant that the sequences of the nucleobase polymer include enough complementarity to hybridize to the single-stranded scaffold. As noted above, in some embodiments, nucleobase polymer can be completely complementary or contain regions of non-complementarity (e.g., mismatches). The exact degree of complementarity will depend upon the desired characteristics of the code region.

In various embodiments, to stabilize the hybridized nucleobase polymer in the non-single-stranded code region, the strands can be crosslinked with an interstrand crosslinker. General classes of interstrand crosslinking agents include, bifunctional DNA alkylating agents (e.g., nitrogen mustards), mitomycin C and analogs thereof (Weidner et al., Biochemistry 1990, 29(39):9225-33); pyrrole derivatives and arylazides (Buchmueller et al., 2000, J Am Chem Soc 125:10850-10861). Non-limiting examples of interstrand crosslinking agents include 5-(aziridin-1-yl)-2,4-dinitrobenzamide, mechloroethamine, chlorambucil, 2,3-bis(hydroxymethyl)pyrrole, dehydroretronecine diacetate (DHRA), 2,3-bis(acetoxymethyl)-1-methylpyrrole (BAMP), dehydromonocrotaline, dehydroretrorsine, sulfidocyclophosphamide (Erickson et al., 1980, Cancer Res 40(11):4216-20; Webb et al., 1986, J Am Chem Soc 108:2764-2765); N,N'-bis(2-chloroethyl)-N-nitrosourea (Pieper et al., 1995, Mol Pharmacol 47(2):290-5); cyanomorpholinoadriamycin (Cullinane and Phillips, Nucleic Acids Res 21(8):1857-1862). Also useful are DNA targeting agents modified to have crosslinking functionalities (see, *e.g.,* Pezzoni et al., Br J Cancer 64:1047-1050; Wurtz et al., Chem Biol 7:153-161; Pastwa et al., 1993, Acta Biochim Pol 40(1):69-71).

To limit the crosslinking of a hybridized nucleobase polymer to the code region, a photoactivable crosslinking agent that binds specifically to the code region can be used. In some embodiments, a photoreactive group is present in the nucleobase oligomer used to form the code region. In other embodiments, the photactivatable crosslinker is present on the defined code region of the scaffold. Exemplary photoactivatable compounds include, among others, arylazide modified nucleobases (Demeshkina et al., 2000, RNA 6:1727-1736; Evans et al., 1986, Proc Natl Acad Sci USA 83(15):5382-6) and psoralen and derivatives thereof (Elsner et al., 1985, Anal Biochem 149(2):575-81; and Hartley et al., 2004, Cancer Res 64: 6693-6699). Other suitable photoactivatable crosslinkers will be apparent to the skilled artisan. Following hybridization of the nucleobase polymer to a defined code region on the scaffold, photoactivation crosslinks the two strands of the code region.

In various embodiments, the coded molecule can be formed in a reaction compartment, which refers to a partitioned or discrete region or volume of a reaction mixture. The reaction compartments form a discontinuous phase of a reaction mixture and the reaction mixture external to the reaction compartments can comprise a continuous phase. At least one or more or all reaction components, such as the scaffold, of a reaction mixture can be substantially sequestered in a reaction compartment. Therefore, in some embodiments, reactions substantially proceed or can be substantially confined within reaction compartments or, in other embodiments, the discontinuous phase in comparison to the continuous phase of a reaction mixture.

In these embodiments, the single-stranded scaffold is within the reaction compartment and can be trapped within the compartment. However, nucleobase polymers used to form the non-single-stranded code regions can be permeable and capable of diffusing in and out the reaction compartment. Thus, in some embodiments, a method for forming a coded molecule can comprise contacting a reaction compartment (*e*.*g*., a microcapsule) with a first nucleobase oligomer in which the reaction compartment comprises a single-stranded nucleobase polymer scaffold, and hybridizing the first nucleobase oligomer to a first defined sequence on the scaffold to form a first non-single-stranded code region.. The method further comprises contacting the microcapsule with an interstrand crosslinking agent to crosslink the hybridized nucleobase oligomer to the scaffold, thereby stabilizing the nucleobase oligomer/scaffold structure. Reaction compartments are washed to remove any unhybridized nucleobase polymer. To form a second code region, the reaction compartments can be contacted with a second oligonucleotide in which the second nucleobase oligomer hybridizes to a second defined sequence on the scaffold to form a second non-single-stranded code region on the scaffold. Hybridization of the second nucleobase oligomer can be done concurrently or subsequent to the hybridization of the first nucleobase oligomer to the scaffold. The second nucleobase oligomer can also be stabilized by crosslinking. The steps can be repeated until all of the non-single-stranded code regions have been formed. The coded molecule is released by disrupting the integrity of the reaction compartment.

In some embodiments, to form different coded molecules, the methods for forming the coded molecules can comprise contacting a population of reaction compartments with a first nucleobase oligomer, where the microcapsules comprise a single-stranded nucleobase scaffold, hybridizing the first nucleobase oligomer to a first defined sequence on the scaffold to form a first non-single-stranded code region, and then generating from the population at least a first and second subpopulation of reaction compartments. To form additional non-single-stranded code regions, the first subpopulation is contacted with a second nucleobase oligomer and the second subpopulation contacted with a third nucleobase oligomer. The first nucleobase oligomer hybridizes to a second defined sequence on the scaffold while the third nucleobase oligomer hybridizes to a third defined sequence to generate a second non-single-stranded code region in each subpopulation. In some embodiments, the second and third defined sequences can be of the same sequence or different sequence. In various embodiments in which the second and third defined sequences are the same, the use of nucleobase oligomers made of different nucleobase polymers, such as PNA and DNA, permits distinguishing between each subpopulation of coded molecules present in the reaction compartments. As above, interstrand crosslinking agents can be used to stabilize the hybridized nucleobase oligomers on the scaffold. By repeating the process of forming subpopulations following synthesis of each non-single-stranded code region and hybridizing nucleobase oligomers to each desired non-single-stranded code region, a large number of differently coded molecules can be generated. However, in each reaction compartment, the coded molecules will have the same ordered code regions, thereby generating substantially identical signal pattern when analyzed through a nanopore.

Thus, in some embodiments, the method of forming a coded molecule comprises contacting a population of reaction compartments with a first nucleobase oligomer in which the reaction compartments comprise single-stranded scaffold. The first nucleobase oligomer hybridizes to a first defined sequence on the scaffold to form a first non-single-stranded code region. The population of reaction compartments is used to generate a plurality of subpopulations of reaction compartments, and each subpopulation contacted with a second nucleobase oligomer that hybridizes to a second defined sequence on the scaffold to form a second non-single-stranded code region. In these embodiments, the second nucleobase oligomer used is different for each subpopulation, either in sequence to which it binds or in the structural characteristics of the nucleobase oligomer.

Various forms of reaction compartments can be used to synthesize the coded molecules in the manner described. In some embodiments, a reaction compartment can be an aqueous compartment of an inverse emulsion. "Inverse emulsion" and "water-in-oil emulsion" ("W/O") as used herein refers to a colloidal composition comprising an aqueous liquid distributed as droplets in a hydrophobic liquid. Thus, in a reaction mixture comprising an inverse emulsion, the reaction compartments can be aqueous droplets comprising the discontinuous phase, which are distributed in a hydrophobic continuous phase. "Microemulsion" are used herein refers to an inverse emulsion in which an aqueous droplet can have an external and/or internal diameter from 1 µm to about 500 µm, from about 1 µm to about 300 µm, from about 1 µm to about 200 µm, from about 10 µm to about 100 µm, and/or from about 25 µm to about 75 µM. In some embodiments, an aqueous droplet of a microemulsion can have a volume from about 0.5 µm³ to about 4,000,000 µm³, from about 500 µm³ to about 500,000 µm³, from about 8,000 µm³ to about 200,000 µm³. However, the skilled artisan will appreciate that larger and smaller droplets also can be contemplated.

The composition of the continuous and discontinuous phases of an inverse emulsion can be selected by those skilled in the art. As described above, a continuous phase can be hydrophobic solution, including, but not limited to, an oil (e.g., mineral oil, light mineral oil, silicon oil) or a hydrocarbon (*e*.*g*., hexane, heptane, octane, nonane, decane), and the like. In contrast, the discontinuous phase can be an aqueous solution that provides conditions suitable for formation of the coded molecule. The composition of the various phases is selected to provide a suitable emulsion under the conditions selected for carrying out the reaction in the aqueous compartments. Therefore, "suitable emulsion" refers to an emulsion that does not substantially degrade, collapse and/or in which the aqueous droplets do not substantially coalesce under the reaction conditions. Therefore, in some embodiments, an emulsion can be suitable for carrying out reactions at various reaction conditions, with variable factors such as temperatures, pH, ionic strength, hybridization conditions, etc., in the presence of various reaction components (e.g., nucleic acids, proteins, enzymes, catalysts, products, by-products, labels, etc.).

In some embodiments, the emulsion can comprise compositions or compounds that modify the emulsion's stability. Such compounds can be amphipathic and therefore comprise hydrophobic and hydrophilic groups, where the hydrophilic group can be polar and/or positively and/or negatively charged. General classes of amphipathic compounds include but are not limited to proteins, polypeptides, and surfactants, such as, detergents and emulsifiers, all of which can be used alone or in any combination. For example, an amphipathic compound can be a protein or polypeptide (e.g., albumin), lecithin, sodium oleate, glycolic acid ethoxylate oleyl ether, 4 (1 aminoethyl)phenol propoxylate, glycolic acid ethoxylate 4 tert-butylphenyl ether, glycolic acid ethoxylate oleyl ether, sodium dodecyl sulfate, 3 [(3 cholamidopropyl)dimethylammonia]-1 propanesulfonate, n dodecyl-β D maltoside (lauryl-β D maltoside), n octyl-β D glucopyranoside, n octyl-β D thioglucopyranoside (OTG), 4 (1,1,3,3-tetramethylbutyl)phenol polymer, N lauroylsarcosine, polyethylene-block-poly(ethylene glycol), sodium 7 ethyl-2 methyl-4 undecyl sulfate, glycolic acid ethoxylate lauryl ether, Atlox® 4912, Tween® 20, Tween® 80, sorbitan monooleate (Span 80), Triton® X 100, Triton® X 114, Brij® 35, Brij® 58, 3 [(3 cholamidopropyl)-dimethylammonio]-1 propane-sulfonate (CHAPS), Nonidet P 40 (NP 40). For further description of these and/or other amphipathic compounds and methods of use in emulsions see, *e.g.,* Emulsions: Theory and Practice, 3rd Ed., 1991, Oxford University Press; Encyclopedia of Emulsion Technology: Basic Theory Vol. 1-IV,, Becher ed., Marcel Dekker Inc., years 1983-1996; Holmberg, Surfactants and Polymers in Aqueous Solutions, 2nd ed., 2002, Jolm Wiley & Sons; Emulsions and Emulsion Technology, Lissant ed., Marcel Dekker Inc., years 1974- 1984; and Handbook of Industrial Surfactants (ISBN 1890595209).

Methods of making inverse emulsions are known in the art and include but are not limited to drop wise addition of an aqueous solution comprising a reaction mixture to a stirred hydrophobic phase, where the hybrophobic phase optionally comprises one or more amphipathic compounds. (see, *e.g.,* Emulsions: Theory and Practice, supra; Encyclopedia of Emulsion Technology: Basic Theory Vol. 1-IV, supra*;* Dressman et al., 2003, Proc. Natl. Acad. Sci. USA 100(15):8817-22 (Epub 2003 Jul 11); Ghadessey et al., 2001, Proc. Natl. Acad. Sci. USA 98:4552-7; Griffiths et al., 2003, EMBO 22:24-35; *Emulsions and Emulsion Technology, supra;* Nakano et al., 2003, J. Biotechnol. 102(2):117-24; Tawfik et al., 1998, Nat. Biotechnol 16(7):652-6; U.S. Patent No. 6,489,103; and WO 2002/22869) Emulsion formation can be monitored by various techniques, such as high-resolution ultrasonic spectroscopy, which measures changes in ultrasonic velocity and attenuation that occur as a function of time are indicative of emulsion formation. In other embodiments, the size (e.g., mean droplet diameter), number, and/or composition of the aqueous phase droplets can be analyzed microscopically (Dressman et al., *supra)* or by laser diffraction (Tawfik et al., *supra).*

In other embodiments for constructing the coded molecules, a reaction compartment can be a microcapsule. "Microcapsule" as used herein refers to a hollow device suitable for providing a reaction compartment of a reaction mixture. In various exemplary embodiments of reaction mixtures comprising microcapsules, the continuous phase can be aqueous or hydrophobic and the microcapsules comprise a discontinuous aqueous phase. In some embodiments, a microcapsule can be placed in an inverse emulsion. The external and/or internal diameter of a microcapsule can be from about 50 µm to about 100 µm in diameter. In some embodiments, the external and/or internal volume of a microcapsule can be from about 65,000 µm³ to 550,000 µm³. However, the skilled artisan will appreciate that larger and smaller microcapsules also can be contemplated.

In some embodiments, the contents of a microcapsule and the continuous phase can be in fluidic communication. Therefore, in some embodiments, various molecules (e.g., reaction components such as nucleobase oligomers and crosslinking agents) can pass between a microcapsule and the continuous phase. In some embodiments, fluidic communication can be by size selective passive transport mechanisms. For example, some molecules can pass through a microcapsule wall to and/or from the various reaction mixture phases, such as through a one or more microcapsule pores. "Pore" as used herein in the context of a microcapsule refers to an opening, hole, or perforation in the wall of the microcapsule. The reaction components can be diffused into or out of the microcapasule through concentration gradients or by use of electromotive driving forces (e.g., voltage gradients). Reaction components can also be entrapped within the microcapsule during formation of the microcapsule.

Generally, the pores of a microcapsule can be modified to be selective for the reaction components entering or exiting a microcapsule for constructing the coded molecule. For example, in various embodiments, a polymer solution (e.g., polyacrylamide) can be absorbed to a microcapsule or removed from a microcapsule to decrease or increase pore size, respectively. Selecting the appropriate features of a microcapsule for the uses described herein is within the abilities of the skilled artisan.

Methods of synthesizing microcapsules are known in the art. For example, in some embodiments a molecule to be encapsulated is mixed throughout another material to form a solid microsphere that allows diffusion of certain reaction components. In other embodiments, microcapsules can be produced by synthesizing the capsule wall around various templates (*e.g.*, latex, silica sols, living cells, inorganic spheres, surfactant vesicles, block copolymer vesicles, emulsion droplets, and gas bubbles). In various embodiments, the microcapsule wall can comprise an organic polymer, an inorganic compound, and/or a composite organic/inorganic composite (see, e.g., Meier, 2000, Chem Soc Rev 29:295-303). Non-limiting examples of methods for synthesizing microcapsules include, among others, production by polymerization and self-assembly methods (Discher et al., 1999, Science 284:1143; Huang et al., 1999, J. Amer. Chem. Soc. 121:3805; Holtz et al., 1998, Langmuir 14:1031; Jenekhe et al., 1998, Science 279:1903; Okubo et al., 1998, Colloid Polym Sci 276:638; Sanji et al., 2000, Macromolecules 33:8524; Wendland et al., 1999, J Am Chem Soc. 121:1389; Zhao et al., 1998, J Am Chem Soc 120:4877), photochemical degradation of polysilane shell cross-linked micelles (Sanji et al., 2000, Macromolecules 33:8524-8526), ozonolysis of shell cross-linked micelles with poly-(isoprene)-β-poly(acrylic acid) (Huang et al., 1999, J Am Chem Soc 121:3805), modification of microporous polycarbonate filtration membranes (Parthasarathy et al., 1994, Nature 369:298-301), γ irradiation to initiate the formation of hollow CdS/polystyrene composites (Wu et al., 2004, Langmuir 20:5192-5195), and suspension polymerization (Okubo et al., 1998, Colloid Polym Sci. 276:638-642).

In still other embodiments, microcapsules can be synthesized by a layer-by-layer (LbL) electrostatic assembly process (Caruso et al., 1998, Science 282:1111; Caruso, 2000, Chem Eur J 6:413; Caruso, 2003, Top Curr Chem 227:145-169; Donath et al., 1998, Angew Chem Int Ed 37:2201). Generally, in this process, a compound having a first charge can be layered onto a template and/or preceeding layer having the opposite charge. This process can be repeated by successively adding layers of opposite charge to the previous layer. The number and composition of layers can be selected at the discretion of the practitioner to synthesize a microcapsule have desired properties (e.g., rigidity, flexibility, elasticity, mechanical strength, porosity, permeability, thermal properties, optical properties). Therefore, in some embodiments, the amount of material utilized for each layer can be used to control the porosity of the microcapsule.

The template, where utilized, can be removed from the microcapsule by various methods, as known in the art, including but not limited to temperature (e.g., calcination) and chemical methods. In various embodiments, a template for microcapsule synthesis can be a colloid (e.g., polymer (Caruso et al., *supra*; Donath et al., *supra*), a metal particle (Gittins et al., 2000, Adv. Mater. 12:1947), protein crystals (Caruso et al., *supra*), low molecular weight compounds (Caruso et al., *supra*), a cell (Caruso, 2000, Chem Eur J 6:413; Moya et al., 2000, Macromolecules 33:4538), melamine formaldehyde microparticle, or a polystyrene microparticle. In various exemplary embodiments, the materials used to layer the template include but are not limited to polyelectrolytes (e.g., poly(diallyldimethylammonium chloride (PDADMAC), nanoparticles (*e*.*g*., SiO₂ nanoparticles, and inorganic molecule precursors (e.g., titanium (IV) bis(ammonium lactano) dihydroxide (TALH: [CH₃CH(O-)CO₂NH]₂ Ti(OH)₂)).

In still other embodiments, the microcapsule is a hydrogel microcapsule synthesized by interfacial polymerization in an inverse emulsion (Arshady, 1989, J Microencapsul 6(1):13-28; Makino et al., 1998, Colloids and Surfaces B: Biointerfaces 12:97-104). In some embodiments, the size of a hydrogel microcapsule can be controlled by controlling the droplet size in the inverse emulsion. Capsule wall permeability can be adjusted by changing conditions, *e.g.,* pH. (Nagashima et al., 1998, Colloids and Surfaces B: Biointerfaces 11:47-56, Narita et al., 2003, Langmuir 19:4051-4054).

In still other embodiments, microcapsules can be produced by self-assembly of colloidal particles at the interface of an inverse emulsion. In this method, small latex particles from an acrylic latex assemble around an aqueous emulsion droplet. The latex particles can be locked together by addition of a polycation or by sintering. The space between the assembled latex particles controls the permeability of the microcapsule. Sintering can proceed by raising the temperature slightly above the glass transition temperature of the polymer in the latex. For example, in some embodiments PMMA particles can be sintered in about 5 minutes at 105°C (Dinsmore et al., 2002, Science 298:1006-1009). Because the latex particles assemble at W/O surface to minimize surface energy, an effect generally independent of the latex chemistry, many polymers other than PMMA can be employed.

In further embodiments, a microcapsule (*e*.*g*., polysulfone microcapsules) can be synthesized by liquid-liquid phase separation techniques (Zhao et al., 2004, J. Microencapsulation 21:283-291). In this method, a polymer solution can be brought into contact with a solution in which the polymer is insoluble resulting in polymer precipitation at the interface. (see, *e.g.,* U.S. Patent Nos. 3,943,065; 4,353,888; 5,441,878; 5,492,789; 5,571,415; 5,691,431; 5,733,462; 5,830,960; 5,837,790; 5,885,032; 5,934,839; 5,985,354; 6,013,708; 6,046,293; 6,248,849; 6,387,995; 6,511,749; 6,528,035; 6,528,093; 6,583,251; 6,599,627; and 6,623,764).

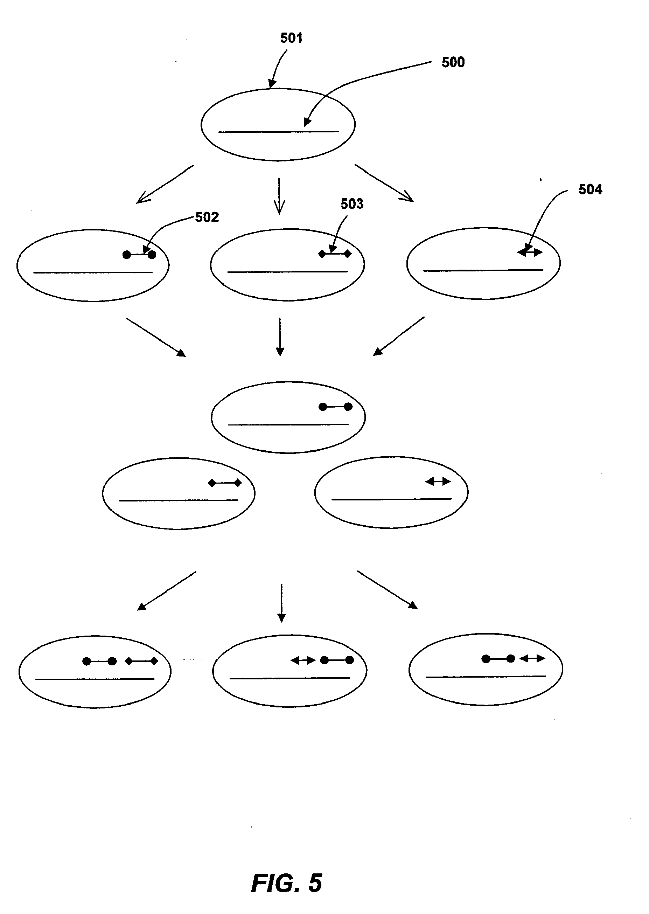

**FIG. 5** illustrates a process for generating a population of coded molecules with at least three non-single-stranded code regions beginning with an emulsion or microcapsule **500** containing a single-stranded scaffold **501.** The population of emulsion droplets with the single-stranded scaffold is divided into three pools, and each pool is contacted with a nucleobase oligomer differing in structure but which hybridizes to the same defined sequence (*i.e.*, a code region) on the scaffold. For instance, in **FIG. 5****,** the first nucleobase polymer **502** comprises a DNA, a second nucleobase polymer **503** comprises a PNA, and the third nucleobase polymer **504** comprises a charged PNA. Each nucleobase polymer is designed to hybridize to the same sequence on the scaffold but display different detectable properties because of the differences in the nucleic acid structure. Optionally, following hybridization, the annealed nucleobase oligomer is crosslinked to the scaffold to stabilize the multistranded structure. The three pools of vesicles with the hybridized nucleobase polymer are mixed together to form a single pool and then redistributed into a second set of three pools. A second set of three nucleobase oligomers, each differing in structure but hybridizing to a defined sequence on a second code region, is added to the microcapsules, allowed to hybridize, and then optionally crosslinked, thereby generating coded molecules with two non-single-stranded code regions. Additional code regions are formed by repeating the process until all non-single-stranded code regions are formed. Each emulsion or encapsulate in the final product contains a population of coded molecules with the same ordered code regions and consequently the same signal pattern when interrogated via translocation through a nanopore. For detecting a target analyte, the population of identical code molecules contained in a single vesicle has attached binding moieties. Attachment can be through a unique sequence or a common sequence that is synthesized onto the scaffold. In some embodiments, a complementary sequence on a binding moiety allows attachment of the binding moiety to the coded molecule.

Variations of the methods above will be apparent to the skilled artisan. For example, a solid support could be used in a manner analogous to the vesicles. Detecting a target nucleic acid can entail synthesis on a single solid support (*i.e.,* a bead) the binding moiety specific for the target analyte along with the scaffold for the coded molecule. The beads are divided into three different pools followed by hybridization of each pool with a different nucleobase polymer to a first code region. The beads are pooled, mixed and then divided again into three different pools. A second set of three nucleobase polymers, each differing in structure but hybridizing to the same defined sequence forming the second code region on the scaffold, is added. The process can be repeated until the desired number of code regions has been established to distinguish each type of coded molecule. Optionally, the hybridized nucleobase oligomer can be stabilized by crosslinking to the scaffold.

In other embodiments, specific combinations of nucleobase polymers that form the non-single-stranded code regions can be mixed and then hybridized concurrently to a single-stranded scaffold. Different combinations of nucleobase oligomers can be used to generate different coded molecules from the same scaffold. This avoids sequential construction of the code regions and allows synthesis of the coded molecule with fewer steps. For example, the following illustrates the preparation of nucleobase polymer mixtures for forming 27 different coded molecules, each molecule having 3 non-single-stranded code regions. In this exemplary embodiment, three different nucleobase polymers that hybridize to a first code region but differ in the detectable property are prepared. Each nucleobase polymer preparation is divided into three aliquots. A second set of nucleobase polymers for forming a second code region is added to each aliquot to generate nine different combinations. This process is repeated for a third set of nucleobase polymers. Each mixture of nucleobase combinations, 27 in all, is reacted with the single-stranded scaffold in separate reactions to produce 27 different coded molecules. Crosslinking can then be used to stabilize the hybridized nucleobase polymers.

In still other embodiments, the coded molecules can be made in reaction compartments formed in a capillary channel, where a reaction compartment is separated from an adjacent reaction compartment by at least one spacing fluid plug. These embodiments are described in U.S. Provisional Application No. 60/710,167, filed August 22, 2005. In these embodiments, the reaction compartment is a liquid segment that fills the maximum inner cross-section of the capillary channel into which the liquid is flowed and which is distinct from adjacent fluid portions formed from an immiscible liquid. Each liquid segment in the channel is referred to as a "slug." The reaction compartment can be an aqueous segment (*i.e.,* a partitioned sample) in the channel distinct from the adjacent non-aqueous segment. In various embodiments, the capillary channel can comprise alternating oil and aqueous slugs. The aqueous slugs acting as reaction compartments can have different or identical nucleobase oligomers and different or identical single-stranded nucleobase scaffolds.

In some embodiments, the scaffold can be attached to an inner wall of the capillary channel, such as by interaction of a biotin moiety and a streptavidin moiety or by other methods known to those skilled in the art. Different scaffolds can be attached to different segments of the capillary channel. In various embodiments, nucleobase oligomers for forming the non-single-stranded code regions can be provided in an aqueous slug that is transported through the capillary channel and which hybridizes to scaffolds attached to the wall of the capillary channel. Each aqueous slug can contain different nucleobase oligomera, thereby allowing formation of the non-single-stranded code regions by transporting various slugs through the capillary channel. Injection pumps can be used to form and transport the aqueous and non-aqueous slugs in the channel.

In other embodiments, the slugs comprise scaffolds, and nucleobase oligomers are added to the slug to form the non-single-stranded code regions. Various permutations of this approach can be used. In some embodiments, the method for forming the coded molecules using a capillary channel can comprise (a) forming at least a first slug comprising a single-stranded scaffold molecule, (b) adding to the first slug a first nucleobase oligomer capable of hybridizing to a first code region, (c) generating a first plurality of slugs from the first slug, and (d) adding to each slug of the first plurality a second nucleobase oligomer, where the second nucleobase oligomer is capable of hybridizing to a second code region. In some embodiments, each nucleobase oligomer added to each slug can be of the same or different type of nucleobase polymer. Use of different nucleobase oligomers that hybridize to the second code region generates a different coded molecule in each slug. In some embodiments, steps (b) through (d) can be reiterated for each slug of the plurality to generate slugs containing coded molecules with increasing number of non-single-stranded code regions. Thus for example, the method can further comprise (e) forming a second plurality of slugs from each slug of the first plurality, (f) adding to each slug of the second plurality a third nucleobase oligomer capable of hybridizing to a third code region. As with the second nucleobase oligomer, the third nucleobase oligomer can be the same or different type of nucleobase polymer for each slug of the second plurality.

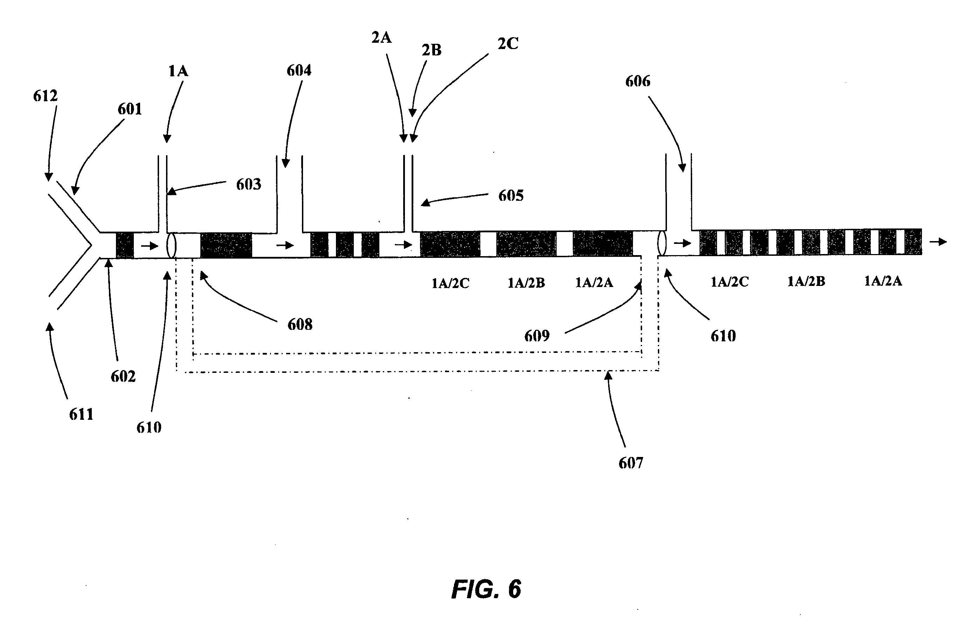

**FIG. 6** illustrates embodiments of a method for forming coded molecules using slugs formed in a capillary channel. A "T" or "Y" junction **601** can be used to generate oil/aqueous slugs in the capillary channel **602.** The aqueous slug comprises a single-stranded scaffold that serves as a platform for forming the coded molecule. A first nucleobase oligomer **(1A)** can be added to the first slug through a first port **603** in fluid communication with the capillary channel. The solution of nucleobase oligomer added to the slug can be set to increase the volume of the slug proportionately so that upon subsequent division of the slug, the slug comprises a dimension equal to the maximum inner cross-sectional dimension. A second port **604** is in fluid communication with the capillary channel and injects a spacing fluid to separate the first slug into a first plurality (synonymously a "population") of slugs, where each slug comprises a dimension equal to the maximum inner cross-sectional dimension. The third port **605** is in fluid communication with the channel and is used to add into each slug of the first plurality a second nucleobase oligomer capable of hybridizing to a second code region on the scaffold. In the illustrated system, at least one of three different nucleobase oligomers **2A, 2B,** and **2C** can be injected into one of the three slugs of the first plurality of slugs. The three slugs in the illustration each have differing second nucleobase oligomers: oligos **1A/2A,** oligos **1A/2B,** and oligos **1A/2C.** A fourth port **606** is used to inject a spacing fluid to form a second plurality of slugs from each slug of the first plurality, and slugs of the second plurality are used in the next step of adding the nucleobase oligomer to form a third non-single-stranded code region. Reiterating the steps of adding nucleobase oligomers to generate additional non-single-stranded code regions followed by injection of spacing fluid produces slugs containing a unique coded molecule.

It is to be understood that systems other than the embodiments described above can be used to form the coded molecule. For instance, a second capillary channel **607** in fluid communication with positions **608** and **609** of the first capillary channel can be used to shunt the first plurality of slugs to a section of the first capillary channel, before the second port **604,** to form the second plurality of slugs by injecting spacing fluid through the second port as each slug of the first plurality passes by. Controllable valves **610** control the flow of slugs through the second capillary channel or through the exit port and also controls entry of the sample into the first capillary channel. In some embodiments, the increased slug volume resulting from added volume of nucleobase polymer solution can be compensated by various measures, such as by corresponding removal of the immiscible liquid in the capillary channel and/or diversion of the slugs into a capillary channel with a larger volume.

In other embodiments, the method can comprise flowing a slug and then changing its flow (e.g., stopping or changing direction of the flow) to process the slugs. Flow can occur in a backward or a forward direction on the capillary channel, or in a loop, thus allowing reagents to be added and the slugs processed similar to the manner described in the various embodiments above.

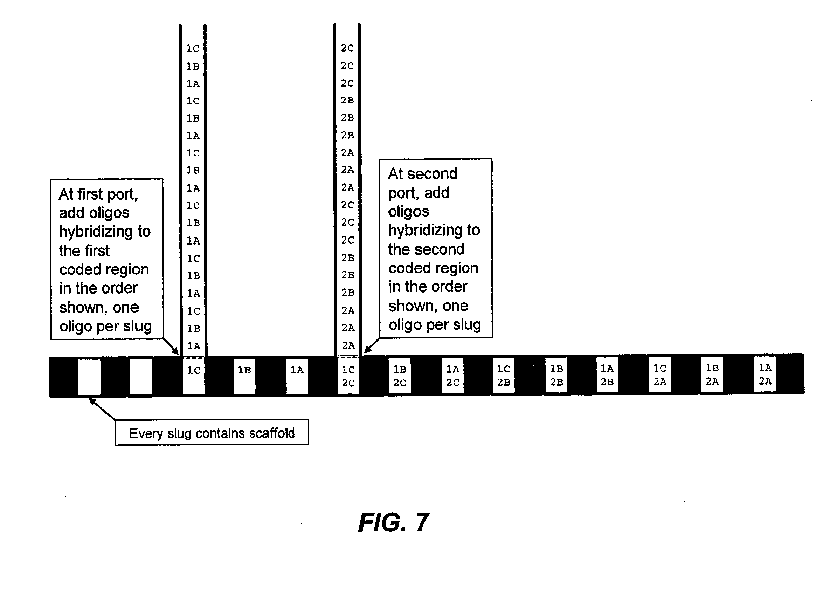

In still other embodiments, the nucleobase oligomers can be added to each slug sequentially to form a coded molecule. In these embodiments, the method can comprise (a) forming at least a first slug in a capillary channel, wherein the first slug comprises a single-stranded scaffold, (b) adding to the first slug a first nucleobase oligomer capable of hybridizing to a first code region on the scaffold. This can be followed by step (c) adding to the first slug a second nucleobase oligomer, wherein the second nucleobase oligomer is capable of hybridizing to the second code region on the scaffold. A plurality of nucleobase oligomers can be added sequentially to generate a coded molecule with a plurality of non-single-stranded code regions. In some embodiments, the nucleobase oligomers added to each slug of a plurality of slugs can be varied to generate coded molecules displaying different signal patterns. Thus, in some embodiments, the first nucleobase oligomer is selected from a first set of nucleobase oligomers that hybridizes to the same sequence on the single-stranded scaffold but that differ as to the type of nucleobase polymer (*e*.*g*., PNA, RNA, or DNA). As additional oligonucletoides are added to the first slug to generate additional non-single-stranded code regions, each nucleobase oligomer can be selected from a set of nucleobase oligomers that hybridize to the same sequence at each defined code region. As noted above, by choosing different combinations of nucleobase oligomers for different slugs, coded molecules that display different signal patterns can be generated from scaffolds of the same sequence. Thus, in some embodiments, the method of making the coded molecule can comprise at least the steps of (a) selecting a first nucleobase oligomer from a first set of nucleobase oligomers capable of hybridizing to a first code region on a scaffold, (b) adding the selected first nucleobase oligomer to a first slug comprising the scaffold, (c) selecting a second nucleobase oligomer from a second set of nucleobase oligomers capable of hybridizing to a second code region on the scaffold, and (d) adding the selected second nucleobase oligomer to the first slug. The selected first nucleobase polymer can be injected via a first port on the capillary channel while the selected second nucleobase polymer can be injected via a second port on the capillary channel. Different ports on the capillary channel can be used to add each nucleobase polymer to each slug as it is transported along the capillary channel (see, *e.g.*, **FIG. 7**). To generate a different coded molecule, the steps of selecting the first and second nucleobase oligomers and adding the selected nucleobase oligomers to the slug are carried out for a second slug, where at least one of the selected nucleobase oligomers is different from that selected for the first slug. Where the coded molecule comprises a plurality of non-single-stranded code regions, the combinations of nucleobase oligomers to be added to a slug can be selected by a computer program to generate a different coded molecule in each different slug.

It is to be understood that in some embodiments, a nucleobase oligomer can be omitted for a particular code region, thereby forming a coded molecule lacking a non-single-stranded code region present in other coded molecules. Thus in some embodiments, the selection from a set of nucleobase oligomers also encompasses the deliberate choice of not selecting any nucleobase oligomer for a specified code region.

In still other embodiments, the combination of nucleobase oligomers used to form the non-single-stranded code region can be added, whole or in part, to the slug concurrently rather than sequentially. In these embodiments, each nucleobase oligomer for each non-single-stranded code region can be selected from a set of nucleobase oligomers, each of which hybridizes to the same sequence on the scaffold. Nucleobase oligomers selected for each of the plurality of non-single-stranded code regions are added to a slug and allowed to hybridize to form a coded molecule in the slug. Different combinations of nucleobase oligomers can be selected and added to each different slug to generate different coded molecules. Thus, in some embodiments, the method of making the coded molecule can comprise at least the steps of (a) forming a first combination of nucleobase oligomers, comprising (i) selecting a first nucleobase oligomer from a first set of nucleobase oligomers, where each nucleobase oligomer of the first set is capable of hybridizing to a first defined region to form a first non-single-stranded code region, (ii) selecting a second nucleobase oligomer from a second set of nucleobase oligomers, where each nucleobase oligomer of the second set is capable of hybridizing to a second defined region on the scaffold to form a second non-single-stranded code region, and (b) adding concurrently the first combination of nucleobase oligomers to a first slug comprising the scaffold. To generate a different coded molecule, a second combination of nucleobase oligomers is formed as in step (a) except at least one of the nucleobase oligomers selected is different from the first combination, and the second combination of nucleobase oligomers added concurrently to a second slug comprising the scaffold. In some embodiments, each nucleobase oligomer combination can be premixed and then injected into different slugs as they pass an injection port in the capillary channel. In other embodiments, the nucleobase oligomers can be injected concurrently into the slug without premixing.

In the embodiments using the capillary channels and slugs, the crosslinking agent can be added to each slug after each nucleobase oligomer is hybridized to the scaffold or after some or all of the nucleobase oligomers have been hybridized to the scaffold. In some embodiments, because there is no mixing of the slugs in the capillary channels, the hybridized nucleobase oligomers can be crosslinked after all of the nucleobase oligomers have hybridized to the scaffold.

Where the code regions comprise detectable tags, they can be incorporated during synthesis of the nucleobase polymer or the scaffold. Phosphoramidite derivatives are available for many detectable tags and can be used during solid phase synthesis of the nucleobase polymers. Another method for incorporating detectable tags onto the code region uses an active ester that can be added to an amino-linked nucleobase polymer, either in solution or in solid phase synthesis. Other methods of attaching tags and labels to nucleobase polymers are described in various references, such as Oligonucleotides and Analogs: A Practical Approach, Ecksterin ed., IRL Press, Oxford, 1991.

Peptides, when part of the coded molecules, can be prepared using standard techniques of organic synthesis. Peptides can be prepared using conventional step-wise solution or solid phase synthesis (see, *e.g.,* Chemical Approaches to the Synthesis of Peptides and Proteins, Williams et al., Eds., CRC Press, Boca Raton Fla. (1997), and references cited therein; FMOC Solid Phase Peptide Synthesis: A Practical Approach, Chan & White, Eds., 2000, IRL Press, Oxford, England, and references cited therein; and Winkler et al., 2005, Bioconjugate Chem 161038-1044.

Linking moieties and methods for attaching the binding moieties and detectable tags to polynucleotides are described in various references available to the skilled artisan. These references include, among others, Oligoizucleotides and Analogs: A Practical Approach, Ecksterin ed., IRL Press, Oxford, 1991; Zuckerman et al., 1987, Nucleic Acids Res. 15:5305-5321; Sharma et al., 1991, Nucleic Acids Res. 19:3019; Gusti et al., 1993, PCR Methods and Applications 2:223-227; U.S. Patent Nos. 4,757,141 and 4,739,004; Agrawal et al., 1990, Tetrahedron Lett. 31:1543-1546; Sproat et al., 1987, Nucleic Acids Res. 15:4837; Nelson et al., 1989, Nucleic Acids Res. 17:7187-7194. Binding moieties can also be attached to a nucleobase oligomer during solid phase synthesis using binding moieties derivatized to form a phosphoramidite (see, *e.g.,* U.S. Patent No. 5,231,191 and 4,997,928).

### 5.3 Nanodevices for Analysis of Coded Molecules

In the present disclosure, detecting the coded molecules is carried out by translocating the coded molecule through a nanopore or nanochannel. As used herein, a "pore" or "channel" refers to an orifice, gap, or conduit of sufficient dimension to allow passage of a single coded molecule. In some embodiments, the channel can be a groove, such as that described in U.S. Patent No. U.S. 6,627,067. In the methods herein, the nanopore or channel is dimensioned for transit of the non-single-stranded region, and thus must be larger than a dimension permissible for passage of a single-stranded nucleic acid but sufficiently discriminating such that a single coded molecule can be detected. Thus, the dimensions of the nanopore will typically depend on the dimensions of the non-single-stranded region and the method used to detect the coded molecule. A coded molecule with a triple-stranded code region can require a nanopore dimension greater than those sufficient for translocation of a coded molecule with a double-stranded code region. Moreover, presence of detectable tags, such as steric modifiers, will also require larger pores or channels than coded molecules lacking such tags. Typically, a pore of about 2 nm or larger will permit passage of a double-stranded nucleic acid molecule. Thus, the nanopore or nanochannel can have has a diameter or gap of about 2 nm or larger, about 3 nm or larger, about 5 nm or larger, to about 10 nm. However, it is to be understood that the pore or channel dimensions can be larger to accommodate coded molecules with the non-single-stranded code regions larger than 10 nm and/or where the device is capable of detecting a single molecule even where the pore or groove can accommodate passage of more than one molecule simultaneously. As an example, for detection based on electron tunneling, the detection region is spatially within a few nanometers of the coded molecule such that the pore or channel is not much larger than the code region itself. However, some detection techniques do not require nanometer proximity of the coded molecule to the detection region and may be capable of detecting a single coded molecule even when the pore, conduit, channel or groove is significantly larger than that required for electron tunneling detection (see, e.g., U.S. Patent No. 6,413,792 and U.S. published application No. 2003/0211502).

Various types of nanopore devices can be used for analyzing the coded molecules. These include, among others, biological nanopores that employ a biological pore or channel embedded in a membrane. Another type of nanodevice is a solid state nanopore in which the channel or pore is made from a fabricated or sculpted solid state component, such as silicon. These and other nanopore devices are applicable for the purposes herein.

### 5.3.1 Biological Nanopores

For detecting the coded molecules, any biological pore with channel dimensions that permit translocation of the non-single-stranded region can be used in analyzing the coded molecules. Two broad categories of biological channels are suitable for the methods disclosed herein. Non-voltage gated channels allow passage of molecules through the pore without a change in membrane potential to activate or open the channel. On the other hand, voltage gated channels require a particular range of membrane potential to activate channel opening. Most studies using biological nanopores have concentrated on α-hemolysin, a mushroom-shaped homo-oligomeric heptameric channel of about 10 nm in length found in *Staphylococcus aureus.* Each subunit contributes two beta strands to form a 14 strand anti-parallel beta barrel. The pore formed by the beta barrel structure has an entrance with a diameter of approximately 2.6 nm that contains a ring of lysine residues and opens into a internal cavity with a diameter of about 3.6 nm. The stem of the hemolysin pore, which penetrates the lipid bilayer, has an average inside diameter of about 2.0 nm with a 1.5 nm constriction between the vestibule and the stem. The dimensions of the stem are sufficient for passage of single-stranded nucleic acids but not double-stranded nucleic acids. Because the coded molecules of the present disclosure have non-single-stranded regions, biological pores having dimensions of the α-hemolysin pore are not suited for the purposes disclosed herein.

A biological nanopore of sufficient dimension for passage of polymers larger than a single-stranded nucleic acid is mitochondrial porin protein, a voltage dependent anion channel (VDAC) localized in the mitochondrial outer membrane. Porin protein is available in purified form and, when reconstituted into artificial lipid bilayers, generates functional channels capable of permitting passage of double-stranded nucleic acids (Szabo et al., 1998, FASEB J. 12:495-502). Structural studies suggest that porin also has a beta-barrel type structure with 13 or 16 strands (Rauch et al., 1994, Biochem. Biophys. Res. Comm. 200:908-915). Porin displays a larger conductance as compared to pores formed by α-hemolysin, maltoporin (LamB), and gramicidin. The larger conductance properties of porin support studies showing that the porin channel is sufficiently dimensioned for passage of double-stranded nucleic acids. Pore diameter of the porin molecule is estimated at 4 nm. The diameter of an uncoiled double-stranded nucleic acid is estimated to be about 2 nm.

Another biological channel that can be suitable for the methods herein are channels found in B *subtilis* (Szabo et al., 1997, J. Biol. Chem. 272:25275-25282). Plasma membrane vesicles made from *B subtilis* and incorporated into artificial membranes allow passage of double-stranded DNA across the membrane. Conductance of the channels formed by B subtilis membrane preparations is similar to those of mitochondrial porin. Although there is incomplete characterization (e.g., purified form) of these channels, it is not necessary to have purified forms for the purposes herein. Diluting the plasma membrane preparations, either by solubilizing in appropriate detergents or incorporating into artificial lipid membranes of sufficient surface area, can isolate single channels in a detection apparatus. Limiting the duration of contact of the membrane preparations (or protein preparations) with the artificial membranes by appropriately timed washing provides another method for incorporating single channels into the artificial lipid bilayers. Conductance properties can be used to characterize the channels incorporated into the bilayer.

In some embodiments, the biological pore can be modified to incorporate a sensing label for sensing the detectable property of the code region, including the sensing of detectable tags incorporated into the code region. Various sensing labels can be used to modify the channel of the biological pore but without significantly affecting the channel dimensions. For example, α-hemolysin has been modified at the pore region by attachment of a short single-stranded nucleotide (via a linker) to a cysteine residue on the hemolysin channel subunit. Pores with modifications to only one of the pore subunits alter the translocation of single-stranded nucleic acids through the hemolysin pore. Single-stranded molecules that hybridize to the attached nucleobase oligomer display current blockades longer in duration than single-stranded nucleic acids that are not complementary to the attached nucleobase oligomer (Howorka et al., 2001, Nature Biotechnol 18:1091-5). Analogously, labels acting as sensors capable of detecting a detectable property of a code region can be attached to the pore of biological channels. As used herein, a "sensing label" refers to a compound or composition that serves to detect a detectable property of the code regions by some interaction with the code region. The interaction need not be direct, and can occur via interactions that occur at a distance (e.g., dipole-dipole interaction). The type of sensing label is dictated by the detectable property of the code region. For instance, where the detectable property of the code region is a fluorescent molecule, the corresponding sensing label can be a fluorescence quencher. In another exemplary embodiment, where the code region comprises a detectable tag that can act either as acceptor or donor member of a FRET pair, the sensing label is the corresponding donor or acceptor. Interrogating the translocating coded molecule with an excitation spectrum of the donor and detecting any emitted light in the emission spectrum of the acceptor identifies the code region containing the complementing member of the FRET pair. Other embodiments of sensing labels will be apparent to the skilled artisan.

For generating the biological nanopores, proteins capable of forming the channels can be isolated from natural sources or made by recombinant methods (Szabo et al., *supra*; Sambrook et al., *supra*; Ausubel et al., *supra*). In other embodiments, isolated plasma membrane preparations can be used as the source of the biological pores. Proteins can be reconstituted into artificial membranes and functional channels detected using standard electrophysiological techniques used to measure single channel activity. Methods and apparatus for incorporating biological pores into artificial membranes are described in U.S. Patent No. 6,267,872.

### 5.3.2 Solid State Nanopores

In other embodiments, analysis of the coded molecules is carried out by translocating the coded molecule through a nanopore or nanochannel fabricated from non-biological materials. Nanopores or channels can be made from a variety of solid state materials using a number of different techniques, including, among others, chemical deposition, electrochemical deposition, electroplating, electron beam sculpting, ion beam sculpting, nanolithography, chemical etching, laser ablation, and other methods well known in the art (see, *e.g.,* Li et al., 2001, Nature 412:166-169; WO 2004/085609). Solid state materials include, by way of example and not limitation, any known semiconductor material, insulating materials, and metals. Thus, the nanopores can comprise without limitation silicon, silicon, silicon nitride, germanium, gallium arsenide, metals (e.g., gold, silver, platinum), metal oxides, and metal colloids.

To control the pore size to nanometer dimensions, various feedback procedures can be employed in the fabrication process. In embodiments where ions pass through a hole, detecting ion flow through the solid state material provides a way of measuring pore size generated during fabrication (see, e.g., U.S. Published Application No. 2005/0126905). In other embodiments, where the electrodes define the size of the pore, electron tunneling current between the electrodes gives information on the gap between the electrodes. Increases in tunneling current indicate a decrease in the gap space between the electrodes. Other feedback techniques will be apparent to the skilled artisan.

In some embodiments, the nanopore is fabricated using ion beam sculpting, as described in Li et al., 2003, Nature Materials 2:611-615. In the described process, a layer of low stress silicon nitride film is deposited onto a silicon substrate via low pressure chemical vapor deposition. A combination of photolithography and chemical etching removes the silicon substrate to leave behind the silicon nitride layer. To form the pore, a focused ion beam (i.e., argon ion beam of energy 0.5 to 5.0 KeV and diameter 0.1 to 0.5 mm) is used to generate a hole in the silicon nitride membrane. By suitable adjustment of the ion beam parameters (e.g., total time the silicon nitride is exposed to the ion beam and the exposure duty cycle) and sample temperature, material can be either removed to enlarge the hole or material added to decrease the hole size. Ion beam bombardment at room temperature and low duty cycle results in migration of material into the hole while bombardment at 5°C and longer duty cycles results in enlargement of the hole. Measuring the amount of ions transmitted through the pore provides a feedback mechanism for precisely controlling the final pore size (Li et al., *supra*). To form a nanopore of useful dimensions, a hole larger than the final desired pore dimensions is made using sculpting parameters that result in loss of the silicon nitride. Subsequently, the size of the pore is adjusted to a dimension suitable for translocation of non-single-stranded code region using sculpting parameters that result in movement of material into the initially formed hole.

In other embodiments, the nanopores can be made by a combination of electron beam lithography and high energy electron beam sculpting (see, e.g., Storm et al., 2003, Nature Materials 2:537-540). A silicon-on-insulator, fabricated according to known methods, is used to form a silicon membrane, which is then oxidized to form a silicon oxide layer. Using a combination of electron-beam lithography and anisotropic etching, the silicon oxide is removed to expose the silicon layer. Holes are made in the silicon by KOH wet etching and the silicon oxidized to form a silicon oxide layer of about 40 nm. Exposure of the silicon dioxide to a high energy electron beam (e.g., from a transmission electron microscope) deforms the silicon dioxide layer surrounding the hole. Whether the initial holes are enlarged or decreased depends on the initial size. Holes 50 nm or smaller appear to decrease in size while holes of about 80 nm or larger increase in size. A similar approach for generating a suitable nanopore by ion beam sputtering technique is described in Heng et al., 2004, Biophy J 87:2905-2911. The nanopores are formed using lithography with a focused high energy electron beam on metal oxide semiconductor (CMOS) combined with general techniques for producing ultrathin films.

In other embodiments, the nanopore is constructed as provided in U.S. Patent No. 6,627,067; 6,464,842; 6,783,643; and U.S. Publication No. 2005/0006224 by sculpting of silicon nitride. Initially, a layer of silicon nitride is deposited on both sides of a silicon layer by chemical vapor deposition. Following addition of photoresist in a manner that leaves a portion of the silicon nitride layer exposed, the exposed silicon nitride layer on one side is removed by conventional ion etching techniques to leave behind a silicon coated with silicon nitride on the other side. The silicon is removed by any number of etching techniques, such as by anisotropic KOH etching, thus leaving behind a membrane of silicon nitride. The thickness of the silicon nitride membrane is controlled by adjusting the thickness deposited onto the silicon. Using electron beam lithography or photolithography, a cavity is produced on one side of the silicon nitride layer followed by thinning of the membrane on the other side of the cavity. Suitable thinning processes include, among others, ion beam sputtering, ion beam assisted etching, electron beam etching, and plasma reactive etching. Numerous variations on this fabrication process, for example, use of silicon nitride layer sandwiched between two silicon layers, can be used to generate different nanopores. As noted above, a feedback mechanism based on measuring the rate and/or intensity of ions passing through the pore provides a method of controlling the pore size during the fabrication process.

In other embodiments, the nanopore is constructed as a gold or silver nanotube. These nanopores are formed using a template of porous material, such as polycarbonate filters prepared using a track etch method, and depositing gold or other suitable metal on the surface of the porous material. Track etched polycarbonate membranes are typically formed by exposing a solid membrane material to high energy nuclear particles, which creates tracks in the membrane material. Chemical etching is then employed to convert the etched tracks to pores. The formed pores have a diameter of about 10 nm and larger. Adjusting the intensity of the nuclear particles controls the density of pores formed in the membrane. Nanotubes are formed on the etched membrane by depositing a metal, typically gold or silver, into the track etched pores via an electroless plating method (Menon et al., 1995, Anal Chem 67:1920-1928). This metal deposition method uses a catalyst deposited on the surface of the pore material, which is then immersed into a solution containing Au(I) and a reducing agent. The reduction of Au(I) to metallic Au occurs on surfaces containing the catalyst. Amount of gold deposited is dependent on the incubation time such that increasing time decreases the inside diameter of the pores in the filter material. Thus, the pore size can be controlled by adjusting the amount of metal deposited on the pore. The resulting pore dimension is measured using various techniques, for instance, gas transport properties using simple diffusion or by measuring ion flow through the pores using patch clamp type systems. The support material is either left intact, or removed to leave gold nanotubes. Electroless plating technique is capable of forming pore sizes from less than about 1 nm to about 5 nm in diameter, or larger as required. Gold nanotubes having pore diameter of about 0.6 nm appears to distinguish between Ru(bpy)2+2 and methyl viologen, demonstrating selectivity of the gold nanopores (Jirage et al., 1997, Science 278:655-658). Modification of a gold nanotube surface is readily accomplished by attaching thiol containing compounds to the gold surface or by derivatizing the gold surface with other functional groups. This feature permits attachment of pore modifying compounds as well as sensing labels, as discussed herein. Devices similar to the cis/trans apparatuses used for biological pores described herein can be used with the gold nanopore to analyze single molecules, .

Where the mode of detecting the coded molecule involves current flow through the coded molecule (e.g., electron tunneling current), the solid state membrane can be metalized by various techniques. The conductive layer can be deposited on both sides of the membrane to generate electrodes suitable for interrogating the coded molecule along the length of the chain, for example, longitudinal electron tunneling current. In other embodiments, the conductive layer can be deposited on one surface of the membrane to form electrodes suitable for interrogating coded molecules across the pore, for example, transverse tunneling current. Various methods for depositing conductive materials are known, including, sputter deposition (i.e., physical vapor deposition), non-electrolytic deposition (e.g., colloidal suspensions), and electrolytic deposition. Other metal deposition techniques are filament evaporation, metal layer evaporation, electron-beam evaporation, flash evaporation, and induction evaporation, and will be apparent to the skilled artisan.

In some embodiments, the detection electrodes are formed by sputter deposition, where an ion beam bombards a block of metal and vaporize metal atoms, which are then deposited on a wafer material in the form of a thin film. Depending on the lithography method used, the metal films are then etched by means of reactive ion etching or polished using chemical-mechanical polishing. Metal films can be deposited on preformed nanopores or deposited prior to fabrication of the pore.

In some embodiments, the detection electrodes are fabricated by electrodeposition (see, e.g., Xiang et al., 2005, Angew. Chem. Int. Ed. 44:1265-1268; Li et al., Applied Physics Lett. 77(24):3995-3997; and U.S. Publication Application No. 2003/0141189). This fabrication process is suitable for generating a nanopore and corresponding detection electrodes positioned on one face of the solid state film, such as for detecting transverse electron tunneling. Initially, a conventional lithographic process is used to form a pair of facing electrodes on a silicon dioxide layer, which is supported on a silicon wafer. An electrolyte solution covers the electrodes, and metal ions are deposited on one of the electrodes by passing current through the electrode pair. Deposition of metal on the electrodes over time decreases the gap distance between the electrodes, creating not only detection electrodes but a nanometer dimensioned gap for translocation of coded molecules. The gap distance between the electrodes can be controlled by a number of feedback processes. In some configurations, the feedback for controlling the distance between the two electrodes uses the potential difference between the two electrodes. As the gap between the electrodes decreases, the potential difference decreases. In other configurations, control of the distance between the two electrodes uses the electron tunneling current across the electrode pair (Li et al, *supra*). As the distance between the electrodes decrease, electron tunneling current increases. Feedback control using electron tunneling appears suitable for fabrication of electrodes with gap distances of about 1 nm or less, while the feedback control based on electrode gap potential allows fabrication of electrodes having gap distances about 1 to about 10 nm. Rate of electrodeposition depends on the electrolyte concentration and the current flowing through the electrodes. Constant current can be used to form layers of metal on the electrodes. In other embodiments, pulses of current can provide precise control over electrode fabrication since pulsed currents can be used to deposit a known number of metal atoms onto the electrodes per each pulse cycle.

In some embodiments, the detection technique is based on imaging charge-induced fields, as described in U.S. Patent No. 6,413,792 and U.S. published application No. 2003/0211502. The methods of fabricating these nanopore devices use techniques employed to fabricate other solid state nanopores. In some embodiments, the field effect detector is made using a silicon-on-insulator that comprises a silicon substrate with a silicon dioxide layer and a p-type silicon layer (doped silicon in which the majority of the charge carriers are positively charged holes). A shallow n-type silicon (doped silicon in which the majority of the charge carriers are negatively charged holes) layer is formed in the p-type silicon layer by ion implantation and addition of an n-type dopant, while another n-type silicon layer that extends through the p-type silicon layer is formed on another region of the silicon-on-insulator. Removal of the silicon substrate and silicon dioxde layers by etching exposes the p-type silicon on the face opposite to the first formed shallow n-type layer. On the newly exposed face of the p-type silicon, a second shallow n-type silicon layer is formed, which connects to the n-type silicon layer that extends through the p-type silicon layer. For interrogating the coded molecules, a nanopore that extends through the two shallow n-type silicon layers and the p-type silicon layer is generated by various techniques, for example by ion etching or lithography (e.g., optical or electron beam). To decrease the nanopore size, a silicon dioxide layer can be formed by oxidizing the silicon. Metal layers are attached to the first formed n-type silicon layer and the n-type silicon layer that extends through p-type silicon, thereby forming the source and drain regions. Detection of the coded molecule, and where suitable, the target analyte, is carried out as further described below.

For analysis of the coded molecules, the nanopore devices can be configured in various formats. In some embodiments, the system comprises a membrane containing the nanopore held between two reservoirs, also referred to as cis and trans chambers (see, *e*.*g*., U.S. Patent No. 6,627,067). A conduit for electron migration between the two chambers allows electrical contact of the two chambers, and a voltage bias between the two chambers drives translocation of the coded molecule through the nanopore. A variation of this configuration is used in analysis of current flow through biological nanopores, as described in U.S. Patent No. 6,015,714, U.S. Patent No. 6,428,959, and Kasianowiscz et al., 1996, Proc Natl Acad Sci USA 93:13770-13773.

Other embodiments of the device above are also disclosed in U.S. application publication no. 2003/0141189. A pair of nanoelectrodes fabricated by electrodeposition is positioned on a substrate surface. The electrodes face each other and have a gap distance sufficient for passage of a single coded molecule. An insulating material protects the nanoelectrodes, exposing only the tips of the nanoelectrodes for the detection of the nucleic acid. The insulating material and nanoelectrodes separate a chamber serving as a sample reservoir and a chamber to which the polymer is delivered by translocation. A cathode and anode electrodes provide a electrophoresis electric field for driving the coded molecule from the sample chamber to the delivery chamber.

The current bias used to drive the coded molecule through the nanopore can be generated by applying an electric field directed through the nanopore. In some embodiments, the electric field is a constant voltage or constant current bias. In other embodiments, the movement of the coded molecule is controlled through a pulsed operation of the electrophoresis electric field parameters (see, e.g., U.S. Patent Application No. 2003/141189 and U.S. Patent No. 6,627,067). Pulses of current can provide a method for precisely translocating one or only a few bases of a coded molecule for a defined time period through the pore and to briefly hold the nucleic within the pore, and thereby provide greater resolution of the electrical properties of the code regions.

The nanopore devices can further comprise an electric or electromagnetic field for restricting the orientation of the coded molecule as it passes through the nanopore. This holding field can be used to decrease the movement of the coded molecule within the pore. Variations in the position of the coded molecule in the nanopore can increase the background noise of the detected signal. For instance, when current blockade is measured, movement of the code region within the pore is likely to result in variations of current flow depending on the position of the coded molecule in the pore. Similarly, where the detection measures electron tunneling current, the current signal is likely to be sensitive to the spatial orientation of the code region relative to the detection electrodes. Movement of the coded molecule, for instance through random Brownian motion, would generate variability in the signal measured, which can create difficulty in assigning a signal pattern to a specific coded molecule. By holding or restricting the orientation of the coded molecule as it translocates through the nanopore, these variations in detected signal can be minimized.

In some embodiments, an electric field that is orthogonal to the direction of translocation is provided to restrict the movement of the coded molecule within the nanopore. This is illustrated in U.S. Publication No. 2003/0141189 through the use of two parallel conductive plates above and beneath the sample plate. These electrodes generate an electric field orthogonal to the direction of translocation of a coded molecule, and thus holds the coded molecule to one of the sample plates. A negatively charged backbone of a DNA, or nucleic acid modified to have negative charges on one strand, will be oriented onto the anodic plate, thereby limiting the motion of the coded molecule. Analogous use of an orthogonal electric field to hold a nucleic acid in a limited orientation for detection is described in U.S. Patent No. 6,627,067. Electrodes positioned to generate an electric field orthogonal to an extended nucleic acid are used to hold the nucleic acid in a groove, where the nucleic acid is interrogated with a probe (e.g., electron tunneling probe). Similar to the control of the electric field for moving the coded molecule through the nucleic acid, the orthogonal electric field can be controlled in regard to the duration and amplitude of the holding field. The electric field used for translocation is coordinated with the electric field used to hold the DNA in a restricted orientation to precisely control the movement of a nucleic acid through the nanopore.

In still other embodiments, controlling the position of the coded molecule is carried out by the method described in U.S. Published Application No. 2004/0149580, which employs an electromagnetic field created in the pore via a series of electrodes positioned near or on the nanopore. In these embodiments, one set of electrodes applies a direct current voltage and radio frequency potential, and a second set of electrodes applies an opposite direct current voltage and radio frequency potential that is phase shifted by 180 degrees with respect to the radio frequency potential generated by the first set of electrodes. This radio frequency quadrupole holds a charged particle (*e*.*g*., nucleic acid) in the center of the field (*i.e.,* center of the pore). Holding the translocating coded molecule in the middle of the nanopore is predicted to reduce the variability of electron flow through a pore and can also provide consistency in current flow measured by electron tunneling. It is suggested that altering the amplitude of the radio frequency quadrupole could also be used to force the coded molecule to one side of the nanopore and slow the rate of translocation through the pore.

### 5.4 Detection Methods

Interrogating each code region as the coded molecule translocates through the nanopore and sensing the detectable property generates a signal for each code region. The combination of the signals from each code region forms a composite signal pattern that identifies the coded molecule. The type of detection method employed will correspond to the property being detected for each code region of the coded molecule. As noted above, the detectable properties include various optical, electrical, magnetic, and steric properties of the code region.

### 5.4.1 Ionic Conductance and Current Blockade

In some embodiments, the detectable property is the effect of the code region on the electrical properties of the nanopore as the coded molecule translocates through the pore. Electrical properties of the nanopore include among others, current, amplitude, impedance, duration, and frequency. As described herein, devices for detecting the pore's electrical properties typically comprise a nanopore in a membrane, either biological or solid state, that separates a cis and trans chambers, which are connected by a conducting bridge. The coded molecule to be analyzed is placed on the cis side of the nanopore in an aqueous solution typically comprising one or more dissolved salts, such as potassium chloride. Application of an electric field across the pore using electrodes positioned in the cis and trans side of the nanopore causes translocation of the coded molecule through the nanopore, which affects the migration of ions through the pore and thereby alters the pore's electrical properties. Current is measured at a suitable frequency to obtain sufficient data points to detect a current signal pattern. The generated signal pattern is then compared to a reference pattern obtained from examination of a single population of coded molecules. Shifts in current amplitude, current duration, and current magnitude define a signal pattern for the coded molecule. The presence or absence of analyte in the sample is then related to the quantity of coded molecule detected in the assay. Measurement of current properties of a nanopore, such as by patch clamp technique, is described in publications discussed above and in various reference works, for example, Hille, B, Ion Channels of Excitable Membranes, 2001, 3rd Ed., Sinauer Associates, Inc., Sunderland, MA.

### 5.4.2 Electron Tunneling Current, Dielectric Constant, and Charge Induced Field Effects

In some embodiments, the detectable property of the code region is quantum tunneling of electrons. Quantum tunneling is the quantum-mechanical effect of transitioning through a classically-forbidden energy state via a particle's quantum wave properties. Electron tunneling occurs where a potential barrier exists for movement of electrons between a donor and an acceptor. To detect electron tunneling, a microfabricated electrode tip is typically positioned about 2 nanometers or less from the specimen (e.g., using a piezoelectric transducer that controls the motion of an electrode formed on a cantilever). At an appropriate separation distance, electrons tunnel through the region between the tip and the sample, and if a voltage is applied between the tip and the sample, a net current of electrons (i.e., tunneling current) flows through the vacuum gap in the direction of the voltage bias. Where the nanodevice employs detection electrodes for measuring tunneling current, the electrodes are positioned proximately to the translocating coded molecule such that there is electron tunneling between the detection electrodes and coded molecule. As further discussed below, the arrangement of the electrodes relative to the translocating coded molecule will dictate the type of electron transport occurring through the coded molecule.

In some embodiments, analysis of the coded molecule involves detecting current flow occurring through the nucleic acid chain (i.e., longitudinally along the nucleic acid chain). For instance, double-stranded DNA is capable of mediating transport of electrons from one end of a short chain to the other end (see, e.g., Murphy et al., 1994, Proc Natl Acad Sci USA 91(12):5315-9). The exact mechanism of electron transfer is unknown, although electron tunneling is given as one explanation for DNA's transport properties. However, the physics underlying electron transport through a double-stranded nucleic acid is not limiting for the purposes herein, and detection of current flowing through the nucleic acid serves to distinguish one code region from another code region, and hence distinguish one coded molecule from another coded molecule. Moreover, the mechanism by which single-stranded and double-stranded nucleic acids conduct electrons are expected to differ because of the absence of significant base stacking in single-stranded nucleic acids, which is theorized to provide overlapping π orbitals for electron transport through a double-stranded DNA. These differences in structure would generate currents that differ significantly between single-stranded and double-stranded nucleic acids and for nucleic acids with sequence differences. For detection of electron flow occurring longitudinally through the coded molecule chain, the detection electrodes are positioned longitudinally to the direction of coded molecule translocation such that there is a gap between the electrodes parallel to the chain of an extended coded molecule. In various embodiments, the detection electrodes can be placed on opposite sides of a layer(s) (e.g. , membrane) separating the two sides of the nanopore, while in other embodiments, the detection electrodes can be positioned within the layer(s) that separate the two sides of the nanopore.

Another mode of electron flow in a nucleic acid is that occurring across the nucleic acid, for example, a direction transverse to an extended nucleic acid chain (e.g., across the diameter of a double-stranded nucleic acid). In a double-stranded nucleic acid, electron transport can occur through the paired bases while in a single-stranded nucleic acid, electron transport can occur through a single unpaired base. Furthermore, differences in the chemical compositions, hydration structures, interactions with charged ions, spatial orientation of each base, and different base pairing combinations will alter the transverse electron transport characteristics, and thus provide a basis for distinguishing code regions that differ in sequence and/or structure of the code region. For detection of electron flow across the coded molecule (i.e., transverse to an extended nucleic acid chain), the detection electrodes are positioned on either side of the nanopore to interrogate the coded molecule across rather than through the nanopore.

In embodiments of longitudinal or transverse detection, the thickness of the electrodes can determine the total number of bases interrogated by the electrodes. For transverse detection, the tips of the detection electrodes can be dimensioned to interrogate a single nucleobase, and thereby possibly obtain single base resolution of each code region. In other embodiments, the dimensions of the detection electrode can be arranged to interrogate more than one nucleobase. Thus, in some embodiments, the number of nucleobases interrogated at any one time can be about 2 or more, about 5 or more, about 10 or more, or about 20 or more depending on the resolution required to detect differences in the code regions.

In some embodiments, the coded molecule can be detected using an electron tunneling probe, such as that used in an electron tunneling microscope. In these embodiments, the electrode tip is rastered across a small region of the sample. As the tip scans the surface, differences in the electron density at the surface of the sample cause corresponding variations in the tunneling current. Changes in tunneling current provide a map of the variations in electron density at the surface of the code region. For the embodiments herein, the coded molecule can be absorbed onto a surface in an extended conformation and then scanned using an electrode tip. In other embodiments, the electrode tip of the electron tunneling microscope is held stationary while the coded molecule is translocated across the tip. A device for translocating a nucleic acid across an electrode probe is described in PCT publication WO 00/79257.

In other embodiments, differences in the structure and spatial orientation of a code region can be detected as differences in capacitance. This type of measurement is illustrated in U.S. application publication no. 2003/0141189. Capacitance causes a phase shift in an applied ac voltage at a defined applied frequency and impedance. As described, phase shift characteristics for each nucleobase is determined for nucleic acids of known sequence and structure, and used as reference standards for identifying individual base characteristics. Nearest-neighbor analysis can permit capacitance measurements extending to more than a single nucleobase.

In some embodiments, the code regions can be detected using a field effect transistor device (FET), such as the n-channel MOSFET (metal oxide FET) device described above (see, e.g., U.S. Patent No. 6,413,792 and U.S. application publication No. 2003/0211502). In these systems, the passage of a charged molecule through the nanopore is believed to induce a change in the charge carrier properties of the p-type silicon layer, thereby altering the electrical conductivity of the p-type silicon layer, which acts as a channel for current flow in a typical MOSFET type device. Typically, the channel in the p-type silicon layer through which the electrons flow is the same type as the source and drain (e.g., n-type). Because each nucleobase residue or combination of nucleobase residues in a nucleobase polymer has different charges associated with it, a particular current profile can be obtained for different code regions. The profile can have a characteristic waveform (e.g., shape, magnitude, and frequency), which can be "imaged" as the coded molecule is scanned through the nanopore.

### 5.4.3 Plasmon Resonance and Raman Spectroscopy

In some embodiments, the code regions can be detected based on the Raman effect, which is the inelastic scattering of photons by molecules. Most of scattering light is elastic and thus the majority of scattered light has the same frequency and wavelength of the incident light. Inelastic scattering is a shift in the wavelength of scattered light (Raman scattering) due to a change in the vibrational, rotational, and electronic energy of a molecule. Thus, Raman spectroscopy detects intrinsic properties of the target molecule. Generally, a sample is interrogated by a light source (e.g., a laser) of known wavelength and polarization, which is directed to the sample by a focusing lens. Scattered light is collected by the focusing lens, filtered to eliminate wavelengths close to the wavelength of the incident light source, and the scattered light of wavelengths in the desired spectral window corresponding to the inelastically scattered light detected. Various Raman spectroscopic techniques are known in the art, including, by way of example and not limitation, surface enhanced Raman spectroscopy (SERS), resonance Raman scattering, coherent anti-Stokes Raman scattering, Raman microscopy, and UV Raman microscopy.

An exemplary Raman technique for sensing the detectable property of the code regions is SERS, an exemplary method of which is described in U.S. Patent No. 5,322,798. This technique relies on enhanced Raman scattering of a compound absorbed onto a surface due to an increased electromagnetic field produced by formation of a charge transfer complex between the surface and molecule being detected. SERS is believed to be dependent on having a surface roughness of characteristic size of about 10 to about 100 nm and can be made of a material whose dielectric constant satisfies a certain resonance condition. Surfaces formed of silver or gold significantly enhance Raman scattering, although other metals such as copper also display enhancement properties. A variation on the surface enhanced plasmon resonance technique employs plasmon resonant particles positioned on a scanning tip to enhance the electromagnetic field near the sample attached to a surface (see, *e*.*g*., U.S. Patent No. 6,002,417; U.S. published application no. 2005/0084912, and WO2004/090505). In these techniques, a probe is designed to have a layer of suitable plasmon resonant material (e.g., silver, gold, platinum, aluminum, or copper). The plasmon resonant surface cna have a single layer of metal or have multiple layers. A beam of light is focused onto the probe, and upon absorbing a photon, the plasmon resonant particle creates a dipole field, which increases the polarization and dipole moment of the molecule near the plasmon resonant particle. In turn, Raman scattered light induces a dipole field in the plasmon resonant particle themselves, further enhancing the Raman scattering effect. To enhance the electromagnetic field near the sample, U.S. application publication no. 2005/0084912 describes plasmon resonant particles arranged symmetrically about a central axis to increase the electromagnetic field at the axis point or at a defined distance from the symmetrically arranged plasmon resonant particles. For scanning a sample, several systems are described. In one system, a cantilever holding the plasmon resonant particles can be positioned proximate to a sample deposited onto a plasmon resonant surface until maximal enhancement of the electromagnetic field is obtained. Translational movement of the sample or the cantilever probe can be used to scan the molecule on the substrate surface, and thereby measure the structural and chemical properties of the compound in question. A variation of this detection method uses a probe in which a nanopore is present at the central axis of the symmetrically arranged plasmon resonant particle probe. Translocation of a polymer (*e*.*g*., DNA) through the nanopore is driven by an electric field in the manner disclosed above and the inelastically scattered light detected by the optical lens on the probe.

### 5.4.4 Optical Detection

In other embodiments, coded molecules can be detected using spectroscopic techniques based on sensing of absorption and/or emission of light. Devices typically comprise an excitatory light source and a photodetector. Light sources for interrogation of the code regions include, among others, gas lasers, solid state lasers, semi-conductor lasers, and light emitting diodes. These can encompass cavity surface emitting lasers, edge-emitting lasers and surface emitting lasers. Detectors for detecting an optical signal include photodiodes, avalanche photodiodes, photomultiplier tubes, phototransistors, silicon photodiodes, charge coupled devices, charge injection devices, hybrid detectors (e.g., charge coupled photodiodes), and complementary metal oxide semiconductor (CMOS). In some embodiments of solid state nanopores, light source and detector can be integrated in a single apparatus. For instance, the nanopore, light source and detector can be placed on a silicon-on-insulator CMOS process (see, e.g., U.S. Patent No. 6,117,643). U.S. Patent No. 6,743,581 describes an integrated circuit constructed using CMOS technology, where a light emitting diode light source and an array of photodiode detectors are contained within a single chip.

Since analysis based on translocation through nanopores examines a single coded molecule, where a code region can have one or more detectable tags, single molecule optical detection techniques can be employed. Detection of single fluorescent molecules are described in Shera et al., 1990, Chem. Phys. Lett. 174:553-7; Enderlein et al., "Fluorescence detection of single molecules applicable to small volume assays," in Microsystem technology: A powerful tool for biomolecular studies," Kohler et al. Eds, Birkhauser, Basel; and Keller et al., Anal Chem 74:316-24A (1999). Detection of emitted photons from single fluorophores typically uses photon counting in combination with fluorescence correlation spectroscopy (FCS), a technique in which spontaneous fluorescence intensity fluctuations are measured in a microscopic detection volume of about 1 femtoliter defined by a tightly focused laser beam. Because excitation is limited to a minute volume, FCS is capable of measuring changes in fluorescence intensity with low background noise. Repetitive excitation of the molecule enhances signal detection. The emitted fluorescence photon bursts can be detected using a microchannel plate photomultiplier based single-photon counter, which can have filtering to remove elastically and inelastically scatter light (Castro et al., 1992, Anal Chem 65:849-852).

In some embodiments, the optical signal (e.g., light scattering, fluorescence, etc.) can be detected using near field scanning optical microscopy (NSOM). In these embodiments, an optical fiber probe scans the sample and detects the optical properties of the sample. The probe has an opaque material covering its surface, except for a small aperture at the tip, which is positioned over a surface at a height above the surface of a few nanometers. The light, usually a laser source, is emitted through this aperture. The aperture also measures light absorption, polarization, transmission, reflection, or fluorescence from the sample. Although the resolution of optical microscopes is limited by the wavelength of light, placing a light point source at a distance less than the wavelength of the light as in NSOM appears to improve resolution by an order of magnitude (see, e.g., D.W. Pohl, "Scanning near-field optical microscopy," in Advances in Optical and Electron Microscopy 12, C.J.R. Sheppard and T. Mulvey, Eds., Academic Press, London, 1990; and Hwang, J. et al., 1998, Proc SPIE, Laser Techniques for Surface Science III 3272:93). Reflection mode is typically for highly scattering and opaque samples. In some embodiments, NSOM can also be used in conjunction with fluorescence or Raman scattering to detect the code regions on the coded molecules.

### 5.4.5 Fluorescence Resonance Energy Transfer and Fluorescence Quenching

In some embodiments, the code regions are detected based on fluorescence resonance energy transfer (FRET), a nonradiative transfer of energy from one fluorophore (the donor) to another (the acceptor) via a dipole-dipole interaction. Upon excitation of the donor, energy transfer to an acceptor molecule results in emission of light at a different wavelength. Spatially, the donor and acceptors must be in proximity of about 1 to about 10 nm to undergo FRET, and the acceptor absorption spectrum must overlap the emission spectrum of the donor. FRET is not mediated by photon emission and does not require the acceptor to be fluorescent, although most applications of FRET use fluorescent donor and acceptor molecules. If the acceptor is also fluorescent, the transferred energy can be emitted as a fluorescence characteristic of the acceptor. If the acceptor is not fluorescent, the energy is lost through equilibration with solvent. Energy transfer typically results in quenching of donor fluorescence, a reduction of the fluorescence lifetime, and a corresponding increase in acceptor fluorescence emission. When the donor and acceptors are different, FRET is detectable by the appearance of the acceptor fluorescence or by quenching of donor fluorescence. When the donor and acceptor are the same, FRET is detectable by the resulting fluorescence depolarization. FRET is strongly dependent on the distance separating the donor and acceptor and their orientation to one another. Generally, the efficiency of the energy transfer process varies in proportion to the inverse sixth power of the distance separating the donor and acceptor molecules. Consequently, FRET provides information on the distance and conformation between a donor and acceptor. Since a coded molecule translocating through a nanopore is in close proximity to the walls of the nanopore, FRET interactions are possible if one of the donor/acceptor pair is localized to the nanopore, as further described below. Suitable donor acceptor pairs will be apparent to the skilled artisan. Exemplary FRET pairs include, by way of example and not limitation, fluorescein and rhodamine, Cy5 and tetramethyrhodamine, Cy3 and Cy5, Cy3B and Cy5, Cy5 and Cy7Q, tryptophan and danysl, BODIPY FL and BODIPY FL, rhodamine and malachite green, phycoerythrin and Cy5, dansyl and octadecylrhodamine, perylene (Pe) and terrylenediimide (TDI), and green fluorescent protein (from *Aequoria Victoria*) and yellow fluorescent protein (YFP). Donor acceptor pairs with high quantum efficiency and large Stokes shift between absorption spectra of the donor and emission spectra of the acceptor can aid in the analysis of the coded molecules. The Forster distance, which is the separation distance where the probability of the energy transfer is 50%, are known to the skilled artisan or can be readily determined for any donor/acceptor pair.

In some embodiments, single molecule fluorescence resonance energy transfer can be used. In these embodiments, a suitable light source, typically a laser, excites a donor molecule. The emitted fluorescence is split into donor and acceptor wavelengths and counted separately (see, e.g., Ha et al., 1996, Proc Natl Acad Sci USA 93:6264-6268; Deniz et al., 1999, Proc Natl Acad Sci USA 96:3670-3675; Ying et al., 2003, Proc Natl Acad Sci USA 100:14629-14634; and Ying et al., 2000, JPhys Chem B 104:5171-5178). In some embodiments, a Fourier transform interferometer spectral imaging device (*e*.*g*., SpectraCube®) can be used to collect the photons and an algorithm employed to quantify each fluorophore by its spectra.

In the analysis of the coded molecule, there are several configurations amenable to FRET type detection of the code regions. In one configuration, the donor or acceptor is attached to the nanopore. For biological pores, a donor or acceptor molecule can be attached to a suitable functional group on the protein pore. The functional group can be one naturally present on the pore or one that can be introduced via a mutation of the natural sequences. For some solid state nanopores with gold particles, such as gold nanotubes, thiol containing donor/acceptor molecules can be readily attached to the gold surface. Attachment to other substrates will be apparent to the skilled artisan. The other member of the FRET pair is attached to one or more code regions on the coded molecule. Translocation through the nanopore brings the code region containing one member of the FRET pair within close proximity to the corresponding member of the FRET pair on the nanopore. Excitation within the limited volume of the nanopore via a focused light source initiates FRET interaction, which can be detected, such as by single photon counting techniques.

Another configuration using FRET is to have a donor molecule on a first code region adjacent to a second code region, where the second code region is modified to contain an acceptor molecule. Presence of adjacent code regions on a coded molecule is identified by detection of FRET at the appropriate excitation and emission spectra. Other variations of FRET will be apparent to the skilled artisan using the guidance provided herein.

In some embodiments, the detection method is fluorescence quenching, which is a reduction in the fluorescence quantum yield without a change in the fluorescence emission spectrum of the fluorescent molecule. Fluorescence quenching can arise in the context of two different fluorescent molecules, such as quenching observed in FRET, and in the context of two of the same fluorescent molecules in close proximity (i.e., self-quenching). Fluorescence quenching also occurs where there is a fluorescent molecule in close proximity to a non-fluorescent dye molecule that absorbs the energy of the excited fluorescent molecule, thereby reducing the quantum yield of the fluorescent molecule. Exemplary non-fluorescent quencher dyes include as non-limiting examples dabcyl, Black Hole Quenchers®(*e*.*g*., BHQ®-0 BHQ®-1, BHQ®-2, and BHQ®-3), and QST dyes (*e*.*g*., QSY-7, QSY-9, QSY-21, and QSY-35). Others are described in WO 03/019145 and U.S. Patent Nos. 6,790,945, 6,727,356, and 6,790,945.

The configurations for detecting the code regions based on fluorescence quenching can use those illustrated for FRET based assays. Thus, in some embodiments, the fluorescent molecule can be attached to the nanopore while the quencher is part of a code region. Fluorescence quenching is expected to occur as the quencher is brought in proximity to the fluorophore on the nanopore by translocation of the coded molecule through the nanopore.

### 5.4.6 Atomic Force Microscopy

In other embodiments, the detection method is atomic force microscopy (AFM). AFM typically uses a cantilever with a microfabricated tip, which is brought in close proximity to the surface of a sample. The force between the tip and the sample leads to a deflection of the cantilever, the magnitude of which is measured using a laser spot reflected from the top of the cantilever onto a segmented photodiode that magnifies small cantilever deflections into large changes in the relative intensity of the laser light. To prevent the tip from breaking, a feedback mechanism is employed to adjust the tip-to-sample distance and keep the force between the tip and the sample constant. Several approaches exist for scanning a sample via AFM. In some embodiments, the sample is mounted on a piezoelectric tube, which can move the sample in the z direction for maintaining a constant force, and the x and y directions for scanning the sample. In other techniques, the tip is raster scanned over the sample surface.

For detecting the surface contours of the sample by AFM, the tips can be operated in contact mode, non-contact mode, and dynamic mode. In the contact mode of operation, the force between the tip and the surface is kept constant during scanning by maintaining a constant deflection. In the non-contact mode, the cantilever is externally oscillated close to its resonance frequency, which is altered by the tip-sample interaction forces. Changes in oscillation with respect to an external reference oscillation provide information about the sample's characteristics. In the dynamic mode, the cantilever is oscillated such that it comes in contact with the sample with each cycle. Additional force is then applied to detach the tip from the sample. Schemes for dynamic mode operation include frequency modulation and amplitude modulation. In frequency modulation, changes in the frequency of modulation provide information about a sample's characteristics. In amplitude modulation, also referred to as intermittent contact or tapping mode, changes in the oscillation amplitude yield topographic information about the sample. Additionally, changes in the phase of oscillation under tapping mode can be used to discriminate between different types of materials on the surface. In the various foregoing embodiments, an AFM probe is positioned at the nanopore and the coded molecule probed as it translocates through the nanopore. In other embodiments, the AFM is scanned over a coded molecule stretched on a clean surface.

It is to be understood that although descriptions above relate to individual detection techniques, the ability to identify a coded molecule with a plurality of detectable properties rather than a single property vastly increases the ability to generate coded molecules with characteristic signal pattern. Thus, in some embodiments, a plurality of different techniques can be used to examine a single coded molecule (see, e.g., Kassies et al., J. Microsc. 2005, 217:109-16). Examples of multiple detection modes include, among others, current blockade and fluorescence, electron tunneling and fluorescence, plasmon resonance and current blockade, and electron tunneling and fluorescence energy transfer. Concurrent detection with different detection modes can be used to confirm presence of a code region by correlating the detection time of the resulting signal between different detection modes.

### 5.5 Use of Coded Molecules for Detection of Target Analytes

The coded molecules of the present disclosure are useful in various applications for detecting the presence of target analytes. "Analyte," "target" and "target analyte" refers to any compound or aggregate of interest, natural or synthetic. Non-limiting examples of analytes include a nucleic acid (e.g., DNA, RNA, PNA, etc.), protein, peptide, carbohydrate, polysaccharide, glycoprotein, lipid, hormone, receptor, antigen, allergen, antibody, substrate, metabolite, cofactor, inhibitor, drug, pharmaceutical, nutrient, toxin, poison, explosive, pesticide, chemical warfare agent, biohazardous agent, radioisotope, vitamin, heterocyclic aromatic compound, carcinogen, mutagen, narcotic, amphetamine, barbiturate, hallucinogen, waste product, contaminant or other molecule. Molecules of any size can serve as targets. Analytes are not limited to single molecules, but can also comprise complex aggregates of molecules, such as a virus, bacterium, spore, mold, yeast, algae, amoebae, dinoflagellate, unicellular organism, pathogen or cell. In certain embodiments, cells exhibiting a particular characteristic or disease state, such as a cancer cell, can be target analytes. Virtually any chemical or biological effector in any form can be detected.

In various embodiments, at least one analyte comprises at least one target sequence, which is a nucleobase sequence, including but not limited to at least one genomic DNA (gDNA), RNA (*e*.*g*., mRNA; noncoding RNA, tRNA, siRNA, snRNA), nucleic acid obtained from subcellular organelles (e.g., mitochondria or chloroplasts), and nucleic acid obtained from microorganisms, parasites, or viruses. Furthermore, a nucleic acid analyte can be present in double-stranded form, single-stranded form, or both double-stranded and single-stranded form. Discussions of nucleic acid analytes can be found in, among other places, Current Protocols in Nucleic Acid Chemistry, S. Beaucage, D. Bergstrom, G. Glick, and R. Jones, eds., John Wiley & Sons (1999) including updates through August 2003.; S. Verma et al., 1998, Ann Rev Biochem 67:99-134; Eddy, S., 2001, Nature Rev Genetics 2:919-29.

In other embodiments, the polynucleotide target analyte is associated with a sequence variation within a population. These sequence variations have uses in evolutionary studies, family relationships, forensic analysis, disease diagnosis, disease prognosis, and disease risk. As noted above, in some embodiments, the polynucleotide target analyte is a single nucleotide polymorphism or SNP. In other embodiments, the polynucleotide target analyte is associated with genetic abnormality, including somatic and heritable mutations, non-limiting examples of which are nonsense mutations, missense mutations, insertions, deletions, and chromosomal translocations.

In some embodiments, the target analyte sequence of interest can be an amplicon generated by any suitable amplification technique including, but not limited to PCR, OLA, LCR. RCA, and RT-PCR (see, e.g., U.S. Patent Nos. 4,683,195; 4,683,202; 4,800,159; 4,965,188; 5,075,216; 5,130,238; 5,176,995; 5,185,243; 5,354,668; 5,386,022; 5,427,930; 5,455,166; 5,516,663; 5,656,493; 5,679,524; 5,686,272; 5,869,252 6,025,139; 6,040,166; 6,197,563; 6,297,016; 6,514,736; and European Patent Nos. EP-A-0200362, EP-A-0201184, and EP-A-320308). Amplicons suitable for use in the methods and compositions described herein can be obtained from cells, cell lysates, and tissue lysates.

In still other embodiments, the target analyte comprises a peptide and proteins, natural and synthetic, that is capable of specifically interacting with the binding moiety of the coded molecule. Non-limiting examples of peptides include immunoglobulins, peptide hormones, cytokines, cellular receptors, enzymes. The protein can be part of a pathogen, such as fungi, bacteria, or virus, or be present in isolation from other components.

In various embodiments, the samples to be analyzed can be obtained from various sources. "Sample" is to be used in the broad sense and is intended to include a wide range of environmental sources and biological materials, including compositions derived or extracted from such biological materials. Non-limiting examples of environmental samples include food, water, soil, waste, or air. Exemplary biological samples include, among others, whole blood; red blood cells; white blood cells; buffy coat; hair; nails and cuticle material; swabs (e.g., buccal swabs, throat swabs, vaginal swabs, urethral swabs, cervical swabs, throat swabs, rectal swabs, lesion swabs, abcess swabs, nasopharyngeal swabs, and the like); urine; sputum; saliva; semen; lymphatic fluid; amniotic fluid; cerebrospinal fluid; peritoneal effusions; pleural effusions; fluid from cysts; synovial fluid; vitreous humor; aqueous humor; bursa fluid; eye washes; eye aspirates; plasma; serum; pulmonary lavage; lung aspirates; and tissues, including but not limited to, liver, spleen, kidney, lung, intestine, brain, heart, muscle, pancreas, biopsy material, and the like. Tissue culture cells, including explanted material, primary cells, secondary cell lines, and the like, as well as lysates, extracts, or materials obtained from any cells, are also within the meaning of the term biological sample as used herein. Materials obtained from forensic settings are also within the intended scope of the term sample.

The samples can be used without further processing or processed according to various methods typically used to prepare samples. For instance, samples containing cells or bacteria can be subjected to physical conditions to disrupt the cells and liberate their contents. Non-limiting examples of such techniques include, among others, sonication, pressure, heat, irradiation, and mechanical shearing. Samples can also be treated with detergents, denaturing agents (*e*.*g*., guanidinium chloride), chaotropic salts, and enzymes such as lysozymes, nucleases, glycosidases, etc. Samples can be subjected to further manipulation, such as filtration, chromatography, precipitation, solvent extraction, and derivatization.

For detecting the target analyte, the sample is contacted with the coded molecule under conditions suitable for interaction of the target analyte and the binding moiety. Typically, conditions are chosen that minimize non-specific interactions and stabilize specific complex formation between the target analyte and binding moiety. The conditions will vary depending on the type of analyte and can be readily determined by the skilled artisan. Guidance is provided in various reference works, such as Sambrook et al., Molecule Cloning: A Laboratory Manual, 3rd Ed., Cold Spring Harbor Laboratory Press (2001), Current Protocols in Molecular Biology, Ausubel. F. ed., Greene Pub. Associates (1998) updates to 2005; Current Protocols in Immunology, Coligan et al. eds., John Wiley & Sons (1998) updates to 2005; and Antibodies: A Laboratory Manual, Harlow et al. eds., Cold Spring Harbor Laboratory Press (1988). Factors for consideration include, among others, incubation time, pH, ionic strength, temperature, and divalent ion concentration. For nucleic acid detection, these conditions can be varied to create a level of hybridization stringency that minimizes hybridization of non-complementary sequences while being stable to complementary target sequences.

In various embodiments, where the coded molecule/target analyte complex is capable of translocating through the nanopore, such as a target polynucleotide hybridized to a probe sequence on the coded molecule, the target analyte can be detected directly via translocation through the nanopore. In these embodiments, the target analyte complexed to the coded molecule has a detectable property distinguishable from the signal pattern of the code regions on the coded molecule. In other embodiments, however, the coded molecule with bound target analyte can be isolated from other coded molecules not bound to the target analyte prior to translocation through the nanopore. This can be advantageous where the number of target bound coded molecules is low compared unbound coded molecules.

In other embodiments, where a coded molecule complexed with a target analyte is incapable of being translocated through the nanopore, the coded molecule-target analyte can be isolated and then separated from the bound target analyte through use of a cleavable linker. These methods can comprise contacting a sample with a coded molecule described herein, isolating the coded molecule with bound target analyte from unbound coded molecules, and cleaving the cleavable linker to separate the coded molecule from the target binding moiety. The coded molecule prepared as such can be analyzed via translocation through a nanopore. In these embodiments, isolation of the coded molecule can use techniques generally used by those skilled in the art. For instance, the complex can be isolated by antibodies specific to the target analyte. The antibodies can be bound to a substrate that can be readily isolated, such as a magnetic bead. In other embodiments, the target analytes can be derivatized with a tag for isolating the target analyte, such as a biotin tag, a metal chelating peptide tag, or a nucleic acid sequence tag. An example of such derivatization is amplification of a target sequence using primers labeled with biotin, which can be easily isolated with various forms of avidin (e.g., streptavidin). The isolated coded molecules can be subjected to conditions resulting in cleavage of the cleavable linker, thereby separating the code molecule from the binding moiety.

In other embodiments, only the coded molecule portion can be translocated through the nanopore and the change in signal by pore blockade used as a basis for detecting the target analyte. Reversing the translocating force can be used to remove the coded molecule from the nanopore. In some embodiments, a single coded molecule can be analyzed multiple times by translocating a coded molecule through a nanopore until there is blockage of the pore by an attached non-translocatable moiety, reversing the driving force (e.g., electric field) to retract and remove the molecule from the pore and then translocating the same coded molecule through the nanopore. This process can be repeated as long as the coded molecule is stable to analysis. Multiple scans of the same coded molecule can be used to identify background noise in the signal generated for each coded molecule.

For sensing the code regions, a coded molecule is placed into a nanopore device and then driven into the nanopore using a suitable force, typically a biased electric current. The driving force can be constant or varied in a controlled manner, such as pulses of current. As the coded molecule translocates through the nanopore, each code region is interrogated to sense the detectable property of the code region. The sensing of each individual code region occurs sequentially as the code regions pass through a detection region. By "sensing" or "scanning" refers to the process of evaluating and/or interrogating the detectable property of the code region in an orderly manner. The order of the code regions can be determined, relative to a reference or orientation point, for example but not limited to, a code region, a detectable tag, or a distinguishable sub-pattern of the signal pattern generated by the code molecule. The signal pattern is then compared to a reference set of signal patterns to identity the coded molecule sampled and relate its identity to the corresponding binding moiety and thus the target analyte detected.

In some embodiments, the code regions of a coded molecule are counted to identify the coded molecule, and thereby identify the corresponding binding moiety and presence of a target analyte. In some embodiments, each non-single-stranded code region detected can be counted as a single signal such that counting the number of non-single-stranded code regions provides a method of identifying the coded molecule and its corresponding binding moiety (see, *e*.*g*., U.S. application publication nos. 2003/0165935 and 2005/0118589; and PCT publication WO 03/045,310). In other embodiments, where the coded molecule comprises detectable tags, each of the detectable tag can be counted. Different detectable tags on a coded molecule can be counted separately and either used alone or in combination with other counted tags to identify the coded molecule and corresponding binding moiety. In other embodiments where a single detectable tag contains multiple detectable labels, each label on a single tag can be counted. An exemplary tag of this type is a quantum dot having different optical labels. Count the number of coded molecules of a certain type to detect the target analyte.

In other embodiments, each coded molecule detected can be counted to determine the total number of counts for a particular coded molecule and therefore the number of target molecules detected. Where the assay measures multiple target analytes, such as in a multiplexing format, counting each different coded molecule can provide a quantitative measure of the amount of different target analytes present in the sample. Counting of each coded molecules can further provide information on other aspects of the binding interactions, such as non-specific binding and binding specificity.

### 5.6 Kits

The coded molecules and devices for their analysis can be provided in the form of kits. The kits can comprise a single type of coded molecule for detecting a single target analyte and corresponding reference standards. In other embodiments, the kit can comprise different coded molecules for detecting the presence of multiple target analytes. Kits can further include nanopore devices created on a single chip for detecting the coded molecules. In various embodiments, the kits can also include instructions for proper use of the coded molecules and nanopore devices. Instructions and diagrams can be on any medium, non limiting examples of which include, among others, printed forms, magnetic tape, flash memory, compact disc, and magnetic disks.

## Claims

1. A method of detecting a target analyte, comprising:
(a) contacting a sample with a coded molecule, wherein the coded molecule comprises (i) an ordered plurality of code regions formed on a single-stranded nucleobase polymer scaffold, wherein one or more of the code regions is non-single-stranded, at least one of the code regions is single-stranded, and each code region has a detectable property such that detecting the ordered code regions generates a defined signal pattern, and (ii) a moiety capable of binding to a target analyte, and wherein contacting is under conditions suitable for interaction of the target analyte with the coded molecule;
(b) translocating the coded molecule through a nanopore, wherein the nanopore is dimensioned for passage of the non-single-stranded region;
(c) sensing the detectable property of each code region as the coded molecule translocates through the nanopore and sensing a detectable property of the target analyte complexed to the coded molecule as the target analyte translocates through the nanopore to generate the defined signal pattern;
(d) relating the signal pattern to presence of the moiety; and
(e) determining the presence of the target analyte.

2. A method of detecting a target analyte, comprising:
(a) contacting a sample with a coded molecule, wherein the coded molecule comprises (i) an ordered plurality of code regions formed on a single-stranded nucleobase polymer scaffold, wherein one or more of the code regions is non-single-stranded, at least one of the code regions is single-stranded, and each code region has a detectable property such that detecting the ordered code regions generates a defined signal pattern, and (ii) a moiety capable of binding to a target analyte, and wherein contacting is under conditions suitable for interaction of the target analyte with the coded molecule;
(b) isolating the coded molecule with the bound target analyte from coded molecules not bound to the target molecule and cleaving a cleavable linker to separate the coded molecule from the target binding moiety;
(c) translocating the coded molecule through a nanopore, wherein the nanopore is dimensioned for passage of the non-single-stranded region;
(d) sensing the detectable property of each code region as the coded molecule translocates through the nanopore to generate the defined signal pattern;
(e) relating the signal pattern to presence of the moiety; and
(f) determining the presence of the target analyte.

3. The method of claim 1 or 2 in which the coded molecule comprises a plurality of non-single-stranded code regions.

4. The method of claim 3 in which at least two of the non-single-stranded code regions are adjacent.

5. The method of claim 3 further comprising a single-stranded region separating at least two of the non-single-stranded code regions.

6. The method of claim 3 in which the plurality of non-single-stranded code regions comprises at least a first and second non-single-stranded code regions, where the detectable property of the first and second code region is distinguishable.

7. The method of claim 1 or 2 in which the non-single-stranded code region is multistranded.

8. The method of claim 7 in which at least one or more of the multistranded region is double-stranded.

9. The method of claim 7 in which at least one or more of the multistranded region is triple-stranded.

10. The method of claim 7 in which the coded molecule has a plurality of multistranded regions.

11. The method of claim 10 in which the plurality of multistranded regions comprises at least one double-stranded and at least one triple-stranded region.

12. The method of claim 7 in which the at least one strand of at least one of the multistranded region is a polynucleotide analog or polynucleotide mimetic.

13. The method of claim 7 in which at least one of the multistranded region comprises a detectable tag.

14. The method of claim 13 in which the detectable tag is an electron transfer label.

15. The method of claim 7 in which at least two strands of at least one of the multistranded region are crosslinked.

## Patentansprüche

1. Verfahren zum Nachweisen eines Zielanalyten, das Folgendes umfasst:
(a) Inkontaktbringen einer Probe mit einem codierten Molekül, wobei das codierte Molekül (i) eine geordnete Vielzahl von Coderegionen, die auf einem einzelsträngigen Nukleobasen-Polymergerüst ausgebildet sind, wobei eine oder mehrere der Coderegionen nicht einzelsträngig ist bzw. sind, mindestens eine der Coderegionen einzelsträngig ist und jede Coderegion eine nachweisbare Eigenschaft aufweist, so dass das Nachweisen der geordneten Coderegionen ein definiertes Signalmuster erzeugt, und (ii) einen Molekülteil, der an einen Zielanalyten binden kann, umfasst, und wobei das Inkontaktbringen unter Bedingungen ist, die zur Wechselwirkung des Zielanalyten mit dem codierten Molekül geeignet sind;
(b) Translozieren des codierten Moleküls durch eine Nanopore, wobei die Nanopore zur Passage der nicht einzelsträngigen Region dimensioniert ist;
(c) Erfassen der nachweisbaren Eigenschaft jeder Coderegion, während das codierte Molekül durch die Nanopore transloziert, und Erfassen einer nachweisbaren Eigenschaft des Zielanalyten, der mit dem codierten Molekül komplexiert ist, während der Zielanalyt durch die Nanopore transloziert, um das definierte Signalmuster zu erzeugen;
(d) Inbeziehungbringen des Signalmusters mit dem Vorliegen des Molekülteils und
(e) Bestimmen des Vorliegens des Zielanalyten.

2. Verfahren zum Nachweisen eines Zielanalyten, das Folgendes umfasst:
(a) Inkontaktbringen einer Probe mit einem codierten Molekül, wobei das codierte Molekül (i) eine geordnete Vielzahl von Coderegionen, die auf einem einzelsträngigen Nukleobasen-Polymergerüst ausgebildet sind, wobei eine oder mehrere der Coderegionen nicht einzelsträngig ist bzw. sind, mindestens eine der Coderegionen einzelsträngig ist und jede Coderegion eine nachweisbare Eigenschaft aufweist, so dass das Nachweisen der geordneten Coderegionen ein definiertes Signalmuster erzeugt, und (ii) einen Molekülteil, der an einen Zielanalyten binden kann, umfasst, und wobei das Inkontaktbringen unter Bedingungen ist, die zur Wechselwirkung des Zielanalyten mit dem codierten Molekül geeignet sind;
(b) Isolieren des codierten Moleküls mit dem gebundenen Zielanalyten von codierten Molekülen, die nicht an den Zielanalyten gebunden sind, und Spalten eines spaltbaren Linkers, um das codierte Molekül von dem das Ziel bindenden Molekülteil zu trennen;
(c) Translozieren des codierten Moleküls durch eine Nanopore, wobei die Nanopore zur Passage der nicht einzelsträngigen Region dimensioniert ist;
(d) Erfassen der nachweisbaren Eigenschaft jeder Coderegion, während das codierte Molekül durch die Nanopore transloziert, um das definierte Signalmuster zu erzeugen;
(e) Inbeziehungbringen des Signalmusters mit dem Vorliegen des Molekülteils und
(f) Bestimmen des Vorliegens des Zielanalyten.

3. Verfahren nach Anspruch 1 oder 2, wobei das codierte Molekül eine Vielzahl von nicht einzelsträngigen Coderegionen umfasst.

4. Verfahren nach Anspruch 3, wobei mindestens zwei der nicht einzelsträngigen Coderegionen benachbart sind.

5. Verfahren nach Anspruch 3, das weiterhin eine einzelsträngige Region umfasst, die mindestens zwei der nicht einzelsträngigen Coderegionen trennt.

6. Verfahren nach Anspruch 3, wobei die Vielzahl von nicht einzelsträngigen Coderegionen mindestens eine erste und eine zweite nicht einzelsträngige Coderegion umfasst, wobei die nachweisbaren Eigenschaften der ersten und der zweiten Coderegion unterscheidbar sind.

7. Verfahren nach Anspruch 1 oder 2, wobei die nicht einzelsträngige Coderegion mehrsträngig ist.

8. Verfahren nach Anspruch 7, wobei mindestens eine oder mehrere der mehrsträngigen Regionen doppelsträngig ist bzw. sind.

9. Verfahren nach Anspruch 7, wobei mindestens eine oder mehrere der mehrsträngigen Regionen dreisträngig ist bzw. sind.

10. Verfahren nach Anspruch 7, wobei das codierte Molekül eine Vielzahl von mehrsträngigen Regionen aufweist.

11. Verfahren nach Anspruch 10, wobei die Vielzahl von mehrsträngigen Regionen mindestens eine doppelsträngige Region und mindestens eine dreisträngige Region umfasst.

12. Verfahren nach Anspruch 7, wobei der mindestens eine Strang von mindestens einer der mehrsträngigen Regionen ein Polynukleotid-Analogon oder ein Polynukleotid-Mimetikum ist.

13. Verfahren nach Anspruch 7, wobei mindestens eine der mehrsträngigen Regionen einen nachweisbaren Tag umfasst.

14. Verfahren nach Anspruch 13, wobei der nachweisbare Tag ein Elektronentransfermarker ist.

15. Verfahren nach Anspruch 7, wobei mindestens zwei Stränge von mindestens einer der mehrsträngigen Regionen vernetzt sind.

## Revendications

1. Procédé de détection d'un analyte cible, comprenant :
(a) la mise en contact d'un échantillon avec une molécule codée, la molécule codée comprenant (i) une pluralité ordonnée de régions de code formées sur un échafaudage polymère de nucléobases à simple brin, la ou les régions de code étant non à simple brin, au moins l'une des régions de code étant à simple brin, et chaque région de code ayant une propriété détectable de telle sorte que la détection des régions de code ordonnées génère un motif signal défini, et (ii) une fraction apte à se lier à un analyte cible, et la mise en contact étant dans des conditions appropriées pour l'interaction de l'analyte cible avec la molécule codée ;
(b) la translocation de la molécule codée à travers un nanopore, le nanopore étant dimensionné pour le passage de la région non à simple brin ;
(c) la détection de la propriété détectable de chaque région de code alors que la molécule codée est transloquée à travers le nanopore, et la détection d'une propriété détectable de l'analyte cible complexé à la molécule codée alors que l'analyte cible est transloqué à travers le nanopore pour générer le motif de signal défini ;
(d) l'association du motif de signal à la présence de la fraction ; et
(e) la détermination de la présence de l'analyte cible.

2. Procédé de détection d'un analyte cible, comprenant :
(a) la mise en contact d'un échantillon avec une molécule codée, la molécule codée comprenant (i) une pluralité ordonnée de régions de code formées sur un échafaudage polymère de nucléobases à simple brin, la ou les régions de code étant non à simple brin, au moins l'une des régions de code étant à simple brin et chaque région de code ayant une propriété détectable de telle sorte que la détection des régions de code ordonnées génère un motif de signal défini, et (ii) une fraction apte à se lier à un analyte cible, et la mise en contact étant dans des conditions appropriées pour l'interaction de l'analyte cible avec la molécule codée ;
(b) l' isolement de la molécule codée avec l' analyte cible lié à partir de molécules codées non liées à la molécule cible et le clivage d'un lieur clivable pour séparer la molécule codée à partir de la fraction de liaison cible ;
(c) la translocation de la molécule codée à travers un nanopore, le nanopore étant dimensionné pour le passage de la région non à simple brin ;
(d) la détection de la propriété détectable de chaque région de code alors que la molécule codée est transloquée à travers le nanopore pour générer le motif de signal défini ;
(e) l'association du motif de signal à la présence de la fraction ; et
(f) la détermination de la présence de l'analyte cible.

3. Procédé selon l'une des revendications 1 ou 2, dans lequel la molécule codée comprend une pluralité de régions de code non à simple brin.

4. Procédé selon la revendication 3, dans lequel au moins deux des régions de code non à simple brin sont adjacentes.

5. Procédé selon la revendication 3, comprenant en outre une région à simple brin séparant au moins deux des régions de code non à simple brin.

6. Procédé selon la revendication 3, dans lequel la pluralité de régions de code non à simple brin comprend au moins des première et seconde régions de code non à simple brin, la propriété détectable des première et seconde régions de code pouvant être distinguée.

7. Procédé selon l'une des revendications 1 ou 2, dans lequel la région de code non à simple brin est à brins multiples.

8. Procédé selon la revendication 7, dans lequel au moins l'une des régions à brins multiples est à double brin.

9. Procédé selon la revendication 7, dans lequel au moins l'une des régions à brins multiples est à triple brin.

10. Procédé selon la revendication 7, dans lequel la molécule codée a une pluralité de régions à brins multiples.

11. Procédé selon la revendication 10, dans lequel la pluralité de régions à brins multiples comprend au moins une région à double brin et au moins une région à triple brin.

12. Procédé selon la revendication 7, dans lequel le au moins un brin d' au moins l'une des régions à brins multiples est un analogue polynucléotidique ou un mimétique polynucléotidique.

13. Procédé selon la revendication 7, dans lequel au moins l'une des régions à brins multiples comprend une étiquette détectable.

14. Procédé selon la revendication 13, dans lequel l'étiquette détectable est un marqueur de transfert d'électrons.

15. Procédé selon la revendication 7, dans lequel au moins deux brins d' au moins l'une des régions à brins multiples sont réticulés.
